# EUROPEAN PATENT APPLICATION

(11) **EP 1 834 944 A1**
(43) Date of publication of application: **19.09.2007**
(21) Application number: 06005510.0
(22) Date of filing: 17.03.2006
(51) Int. Cl.: C07C 67/00, C07C 69/02, C07C 69/66, C07D 207/34, C07D 405/06, C07D 319/06, C07F 9/547, C07C 217/28

(54) **Process for preparing C7 intermediates and their use in the preparation on N-substituted pyrrole derivatives**

(71) Applicant: Ratiopharm GmbH, 89079 Ulm (DE)
(72) Inventor: Korostylev, Andrei, Ryazan 390000 (RU); Tararov, Vitali, Moscow (RU); Börner, Armin, 18059 Rostock (DE); König, Gerd, 08056 Zwickau (DE); Bobál', Pavel, 901 01 Malacky (SK); Frantisek, Jaroslav, 61800 Brno (CZ); Stohandl, Jirí, 592 55 (CZ); Denike, Kane, Georgetown, Ontario L7G 5Y4 (CA); Jeker, Nicolas, 4143 Dornach (CH)
(74) Representative: Teipel, Stephan

(57) **Abstract**

The present invention relates to a process for preparing C7 intermediates and their use in the preparation of pyrrole derivatives of a class that is effective at inhibiting the biosynthesis of cholesterol in humans, and more particularly to improved synthetic methods for preparing 3,5-dihydroxy-7-pyrrol-1-yl heptanoic acids. The invention further relates to intermediates in this process.

## Description

The present invention relates to a process for preparing C7 intermediates useful for preparing pyrrole derivatives of a class that is effective at inhibiting the biosynthesis of cholesterol in humans, and more particularly to improved synthetic methods for preparing 3,5-dihydroxy-7-pyrrol-1-yl heptanoic acids. The invention further relates to intermediates in this process.

It is known that certain 3,5-dihydroxy heptanoic acid derivatives are competitive inhibitors of the 3-hydroxy-3-methyl-glutaryl-coenzyme A ("HMG-CoA"). HMG-CoA is a key enzyme in the biosynthesis of cholesterol in humans. Its inhibition leads to a reduction in the rate of biosynthesis of cholesterol. The first HMG-CoA inhibitor to be described is compactin ([1*S-*[1α(*R**), 7β, 8β(2*S**, 4*S**),8αβ]]-1,2,3,7,8a-hexahydro-7-methyl-8-[2-(tetrahydro-4-hydroxy-6-oxo-2*H*-pyran-2-yl)ethyl]-1-naphthalenyl 2-methylbutanoate), which was isolated from cultures of *Penicillium* in 1976. In 1987, lovastatin ([1*S*-[1α(*R**),3α, 7β, 8β(2*S**, 4*S**),8αβ]]-1,2,3,7,8,8a-hexahydro-3,7-dimethyl-8-[2-(tetrahydro-4-hydroxy-6-oxo-2*H*-pyran-2-yl)ethyl]-1-naphthalenyl 2-methylbutanoiate) became the first HMG-CoA reductase inhibitor approved by the Food and Drug Administration (FDA) for treatment of hypercholesterolemia. Both compactin and lovastatin are derived from bacterial cultures. Two other naturally-derived HMG-CoA reductase inhibitors, simvastatin and pravastatin are structurally related to compactin and lovastatin.

In 1987, it was reported in U.S. patent No. 4,681,893 that compounds within a certain class of 3,5-dihydroxy-7-pyrrol-1-yl heptanoic acid (and the corresponding lactones) also were effective at inhibiting the HMG-CoA reductase enzyme. One such compound is [*R*(*R**,*R**)]-2-(4-fluorophenyl)-β,δ-dihydroxy-5-(1-methylethyl)-3-phenyl-4-[(phenylamino)carbonyl]-1H-pyrrole-1-heptanoic acid ("atorvastatin"), which was said to provide surprising inhibition in U.S. patent No. 5,273,995. Atorvastatin later received FDA approval as an adjunct to a low cholesterol diet to reduce elevated levels of total cholesterol, low density lipoprotein cholesterol, apo B and triglycerides and to increase levels of high density lipoprotein cholesterol in patients with hyperlipidemia.

In contrast to compactin, lovastatin, simvastatin and pravastatin, there is no known fermentation culture that produces atorvastatin. It, and other 3,5-dihydroxy-7-pyrrol-1-yl heptanoic acids, must be synthesized by traditional synthetic methods.

A number of processes for the synthesis of 3,5-dihydroxy-7-pyrrol-1-yl heptanoic acids and in particular atorvastatin are known. Some of the processes are concerned with the synthesis of the 3,5-dihydroxy heptanoic acid side chain of the pyrrole ring while others are concerned with the formation of the pyrrole ring.

For example, EP-A-0 330 172 teaches that the pyrrole ring can be formed by the Paal-Knorr reaction between an 1,4-diketone and a primary amine being a precursor of the 3,5-dihydroxy heptanoic acid side chain. The 1,4-diketone already bears those substituents required at the pyrrole ring of atorvastatin and in particular the (phenylamino)carbonyl group required at position 4 of the pyrrole ring.

It is, however, difficult to obtain the required 1,4-diketone comprising the amino carbonyl moiety in good yield and purity. Moreover, even when starting from a commercially available 1,4-diketone precursor comprising the required (phenylamino)carbonyl side chain, it turned out to be difficult, if not impossible, to replace the phenylamino residue by other residues such as alkoxy or aryloxy residues without cleavage of the diketone. This limits the possible variation of the substituents and makes a screening for new compounds for inhibiting the biosynthesis of cholesterol in humans difficult.

Moreover, several approaches have been published to synthesize the C7 side chain on,the 1 position of the pyrrole ring in atorvastatin. For example WO 2005/012246 discloses processes and intermediate compounds for the preparation of statins, particularly atorvastatin. Synthesis of the C7 side chain containing the amino group which is afterwards reacted with a 1,4-diketone to form the pyrrole ring is done via a lactol. EP-A-0 409 281 suggests to first prepare the required 2,3,4,5-substituted pyrrole derivative bearing a C3 substituent on position 1. This C3 substituent is then converted in a C5 substituent and subsequently into the desired C7 substituent. This has, however, the disadvantage that the chain extension has to be carried out on the pyrrole derivative with its various other substituents making the reaction difficult to handle and bearing the risk of a potential partial loss of the valuable pyrrole derivative. In particular during synthesis of the necessary stereoconfiguration of the two hydroxy groups of the C7 side chain after linkage to the pyrrole core there is significant loss of the valuable pyrrole derivative due to low enantiomeric excess synthesis or necessary racemate separation.

Therefore, there is still a need for further methods of synthesizing pyrrole derivatives and in particular intermediates for the preparation of 3,5-dihydroxy-7-pyrrole-1-yl heptanoic acids having HMG-CoA inhibitory activity.

It has now been found that several of the prior art problems can surprisingly be overcome by certain C7 intermediates and the reaction of the C7 intermediates with certain pyrroles to pyrroles having a C7 substituent on position 1. In particular, it has been found that the required C7 intermediate can be synthesized from C5 intermediates in a preparative scale, said C5 intermediates can be synthesized according to the following reaction scheme 1.

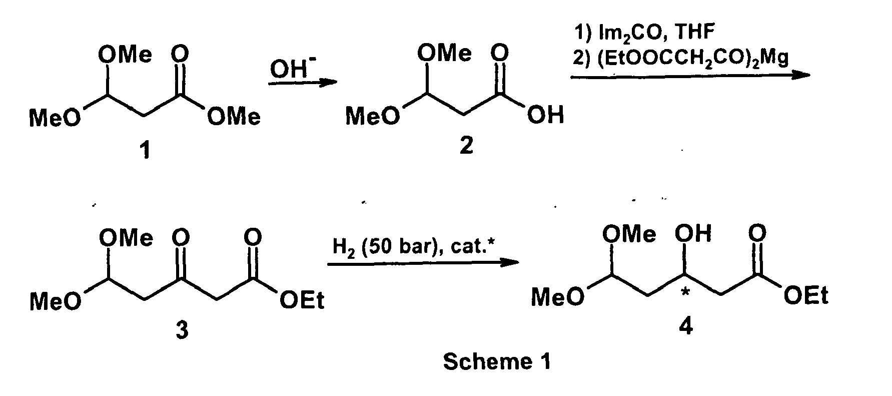

The advantage of this sequence is the high enantioselectivity which can be achieved synthesizing the C5 intermediate.

In particular it was surprisingly found that the reduction of a β-keto ester containing an acetal group can be performed with excellent enantiomeric excess (ee).

Therefore, the present invention relates to a process for the preparation of a compound of the formula I 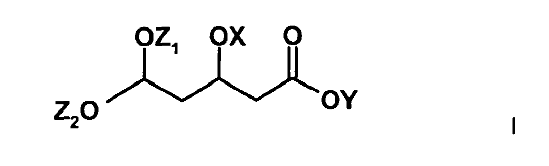 or a salt thereof, wherein Z₁ is a hydroxy protecting group, Z₂ is a hydroxy protecting group, X is H or a hydroxy protecting group, and Y is H or a carboxy protecting group, which process comprises the steps of
a) reacting a compound of the formula II 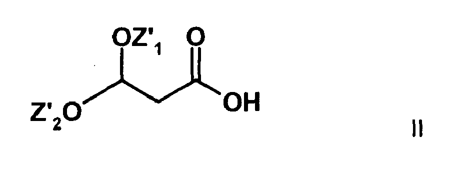 wherein Z'₁ is a hydroxy protecting group and Z'₂ is a hydroxy protecting group, with N,N'-carbonyldiimidazole (Im₂CO) and a compound of the formula III 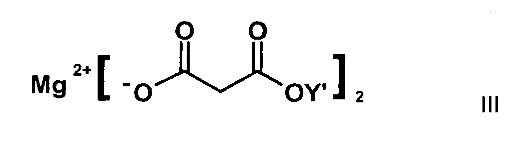 wherein Y' is a carboxy protecting group, to a compound of the formula IV 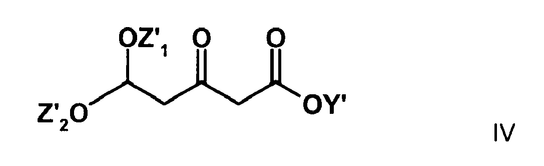 wherein Z'₁, Z'₂ and Y' are defined as above,
b) reducing the compound of the formula IV to obtain a compound of the formula V 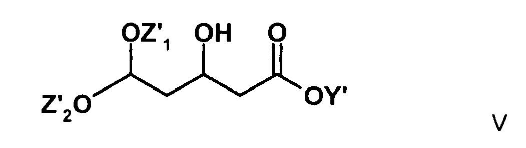 wherein Z'₁, Z'₂ and Y' are defined as above, and
c) optionally deprotecting the hydroxy groups and/or the carboxy group of the compound of the formula V, optionally protecting the free hydroxy group of the compound of the formula V and/or optionally converting the resulting compound into a salt thereof.

The compound of formula I prepared by the process of the present invention is intended as intermediate for the preparation of the C7 side chain of formula VIb for the preparation of pyrrole derivatives. The C7 side chain can be prepared by reacting the compound of the formula I with a compound of the formula XV. Further, the process of the present invention includes modification steps of the C7 side chain including reduction steps to obtain a compound of the formula VIb, which can further be used for the preparation of pyrrole derivatives by reacting with a compound of the formula VII, which is unsubstituted at position 1, to obtain the desired pyrrole derivatives as described in more detail below.

As protecting groups for the optionally protected hydroxy groups and the optionally protected carboxy groups usual protecting groups known to the person skilled in the art may be used. Suitable protecting groups are exemplified in WO 03/044011, the content of which in incorporated by reference herein.

A preferred protecting group for Z₁, Z₂, Z'₁, Z'₂, Z"₁, Z"₂ and Z₅ is a methyl group. Preferred protecting groups for X, X₁, X₂, W₁, Y, Y', Y" and Z₃ are alkyl, aryl and aralkyl, such as straight, branched or cyclic C₁₋₁₀ alkyl, preferably C₁₋₆ alkyl, more preferably methyl, ethyl, iso-propyl or tert-butyl. Aryl can be for example phenyl or naphthyl. Aralkyl can be for example aryl such as phenyl or naphthyl linked via a C₁₋₁₀ alkylene, preferably C₁₋₆ alkylene, such as benzyl.

A preferred protecting group for X₁ and X₂ when forming a ring with the oxygen an intermediate carbon atoms is a 2,2-propyl group linked to both oxygens at the 2-position.

The reduction step (b) in the process of the present invention, which preferably is a hydrogenation step, can be carried out under chiral or achiral conditions. Under achiral conditions a mixture of the two enantiomers of the compound of formula I is obtained. If it is desired to obtain the compound in its chiral form comprising mainly only one of its enantiomers, the reduction step (b) can be carried out under chiral conditions, for example in the presence of a chiral catalyst. Preferably the hydrogenation is carried out under homogenous chiral enantioselective conditions.

Suitable catalysts may be selected from iridium, rhodium and ruthenium complexes, such as Ru((*R*)-BINAP) = (*R*)-2,2'-Bis(diphenylphosphanyl)-1,1'-binapthyl and Ru((*R*)-Tol-BINAP) = (*R*)-2,2'-Bis(di-*p*-tolylphosphanyl)-1,1'-binapthyl, which are preferred.

For example Ru-BINAP catalysts are described in Tetrahedron Lett. 1991, 32, 4163 and WO 95/18784, the content of these documents is incorporated herein by reference.

Preferably the ligand in the Rh, Rn and Ir complexes should be chiral. For example the following ligands can further be used for Rh, Ru and Ir catalyzed asymmetric hydrogenation:
(*R*)-NORPHOS - (2*R*,3*R*)-(-)-2,3-bis(diphenylphosphanyl)-bicyclo[2.2.1]hept-5-ene.
(*R,R*)-CHIRAPHOS = (2*R*,3*R*)-(-)Bis(diphenylphosphanyl)butane
(*R,R*)-DEGUPHOS = (3*R*,4*R*)-(+)-1-Benzyl-3,4-bis(diphenylphosphanyl)pyrrolidine
(*R,R*)-Me-DUPHOS = (-)-1,2-Bis[(2*R*,5*R*)-2,5-dimethylphospholanyl]benzene
(*R*,*R*)-Et-DUPHOS = (-)-1,2-Bis[(2*R*,5*R*)-2,5-diethylphospholanyl]benzene
(*R*,*R*)-DIPAMP = (1*R*,2*R*)-Bis[(2-methoxyphenyl)phenylphosphanyl]ethane
(*R,R*)-bdpch = (1*R*,2*R*)-(*trans*)-1,2-Bis(diphenylphosphanyloxy)cyclohexane
(*S*,*S*)-DIOP = (4*S*,5*S*)-(+)-4,5-Bis(diphenylphosphanylmyrthyl)-2,2-dimethyl-1,3-dioxolane
Ph-β-Glup = Phenyl 4,6-*O*-benzylidene-2,3-bis(*O*-diphenylphosphanyl)-β-D-glucopyranoside.

In the above ligands the configuration is given as example only. It is understood that the skilled person will choose the configuration of the ligand such that the obtained hydrogenated product has the desired configuration.

The hydrogenation can be carried out under usual conditions, for example in the presence of hydrogen at about room temperature, such as 25°C, under elevated pressure for example in the range of 20 - 80 bar, preferably 30 - 70 bar and in particular 40 - 50 bar. The reaction can be carried out in a suitable solvent, preferably a polar protic solvent, in particular a C₁₋₆ alcohol, such as methanol, which is preferred, or ethanol, or an ester, such as ethyl acetate. The reaction can be carried out until hydrogen consumption is finished.

However, when trying to synthesize the required tert-butyl ketoester of the formula IV starting from a compound of formula I using traditional Knoevenagel reaction in the last step, while the desired ketoester was formed it was contaminated with about 15% tert-butyl acetoacetate. No reasonable method of purification was found.

While the mono tert-butyl malonate required in the preparation of the intermediate compound of the formula III is commercially available, this compound is expensive and therefore not feasible for use in a commercial process. Therefore, the inventors tried to obtain the required mono tert-butyl malonate by reaction of Meldrum's acid with tert-butanol according to D.W. Brooks, et al., Tedrahedron Lett., 1984, 25, 4623-26 and S. Masayuki, et al., Tetrahedron Lett., 1997, 38, 4623-26 (see reaction scheme 2). It was found that refluxing of Meldrum's acid in excess of tert-butanol gives the desired mono tert-butyl malonate as the main product but also the cyclic compound depicted in scheme 2 as the side product in a ratio of about 30:1.

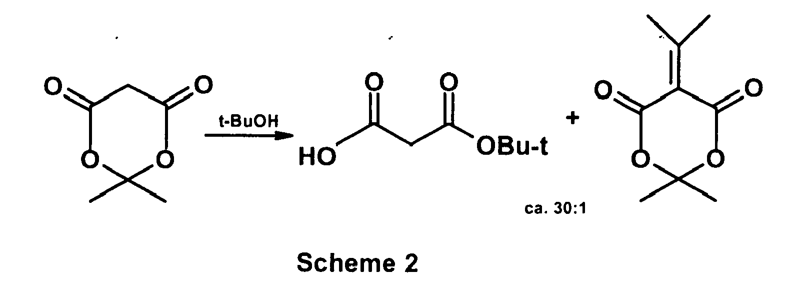

Purification of mono tert-butyl malonate by distillation was unsuccessful since disproportionation of the material to di-tert-butyl malonate and malonic acid was observed.

Surprisingly it was found that a fast purification of mono tert-butyl malonate via its ammonium salt is possible. This salt can be isolated with a yield of 86.7%. Treatment of this salt with hydrochloric acid affords pure (NMR) mono tert-butyl malonate with a yield of 94.3%.

Therefore, the process for the preparation of a compound of the formula XXX 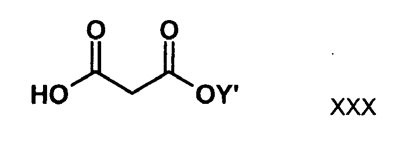 wherein Y' is a carboxy protecting group, which process comprises the steps of reacting Meldrum's acid 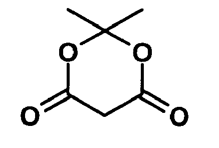 with a compound of the formula Y'OH, wherein Y' is defined as above, and purifying the obtained mono protected malonate via its ammonium salt is advantageously combined with the process of the present invention.

In this process Y' preferably is tert-butyl.

Advantageously this process is combined with the above process for the preparation of a compound of the formula I by converting the compound of the formula XXX into its magnesium salt which is then used as intermediate of the formula III.

In the process of the present invention for the preparation of the compound of the formula I reaction step (a) can be carried out under conditions as described for example in T. Honore, et al., Eur. J. Med. Chem. Chim. Ther., 1978, 13, 429-34.

Surprisingly, several trials of the analysis of the alcohol 4 by gas chromatography on various chiral columns gave no satisfactory resolution of enantiomers. As a convenient alternative, enantiomeric compositions of the product were determined with the help of a chiral derivatising agent XII (Scheme 3). This chlorophosphite is readily available from (*S*)-BINOL in one step (Francio et al., Eur. J. Inorg. Chem. 1999, 8, 1219-1228) and reacts quantitatively and fast with the alcohol 4 to give phosphites XIIa and XIIb. The ³¹P NMR spectrum of both diastereomeric compounds XIIa and XIIb is characterized by signals separated till the base line (δ_{P} 151.4 and 154.6), therefore it is possible to determine precisely the enantiomeric composition of the catalytic hydrogenation product. According to the well established general trend, ^{5a,12b} Ru((*R*)-BINAP)Cl₂ produced (R)-4 as a predominant enantiomer.

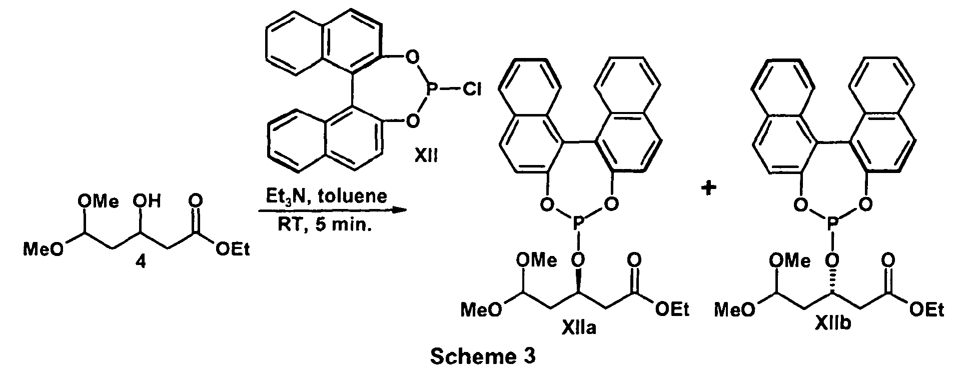

The compounds of the formula I are intended as intermediates for the preparation of the C7 side chain of formula VIb for the preparation of pyrrole derivatives. Preferably the process of the present invention therefore comprises the step of reacting the compound of the formula I with a compound of formula XV 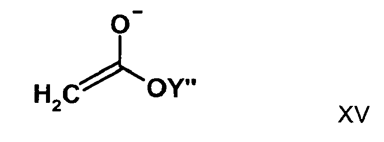 wherein Y" is a carboxy protecting group to obtain a compound of formula VI 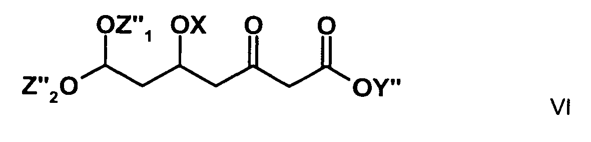 or a salt thereof, wherein Z"₁ is a hydroxy protecting group, Z"₂ is a hydroxy protecting group, X is H or a hydroxy protecting group and Y" is defined as above and optionally deprotecting and/or protecting any protected or unprotected group.

Preferably, according to the present invention a compound of formula VI is further hydrogenated by a process comprising the step of achiral or chiral enantioselective hydrogenation of a compound of formula VI and optionally deprotecting and/or protecting any protected or unprotected group to obtain a compound of formula VIa 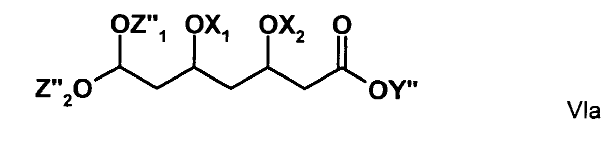 or a salt thereof, wherein Z"₁ is a hydroxy protecting group, Z"₂ is a hydroxy protecting group, X₁ and X₂ can be independent from each other a hydroxy protecting group or X₁ and X₂ taken together may form a ring with the oxygen and intermediate carbon atoms to which they are attached and Y" is defined as above.

One advantage of the process of the present invention is that the C5 chains, having a protected hydroxyl group or a hydroxyl group at the 3-position, can be prepared with excellent enantiomeric excesses. Therefore, the corresponding C7 side chain, which can be produced from the C5 chains by the process of the present invention, can be prepared, when having two protected or unprotected hydroxyl groups at the 3-position and 5-position, in excellent diastereomeric excesses, for example by applying a hydration to a compound of formula VI leading to the syn-product, for example a compound of the formula VIa.

Preferably, according to the present invention the compound of formula VIa is further modified by a process comprising the step of hydrogenation and/or group modification of a compound of formula VIa to obtain a compound of formula VIb 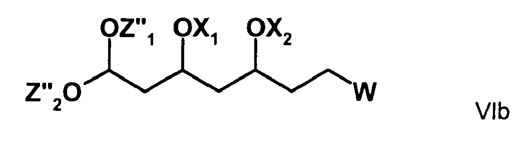 wherein Z"₁ is a hydroxyl protecting group, Z"₂ is a hydroxyl protecting group, X₁ and X₂ can be independent from each other a hydroxyl protecting group or X₁ and X₂ taken together may form a ring with the oxygen and intermediate carbon atoms to which they are attached and W is NH₂, N₃, OH, OW₁ or a leaving group and W₁ is a hydroxyl protecting group.

A leaving group according to the present invention is any residue suitable for rendering the carbon atom of the C7 side chain, which the residue is linked to, receptive for a nucleophilic substitution. Preferred examples are halogens like chloride, bromide or iodide, or tosylate (p-toluene sulfonate). Further equivalent leaving groups and how to introduce those groups is known to the person skilled in the art.

The compounds of the formula VIb are intended as intermediates for the preparation of pyrrole derivatives, for example by substitution or introduction to the 1-position of pyrrole derivatives, which can be substituted at the 2-, 3-, 4- and/or 5-position. Preferably, the process of the present invention therefore comprises the further step of reacting the compound of the formula VIb, wherein W is a leaving group, with a compound of the formula VII 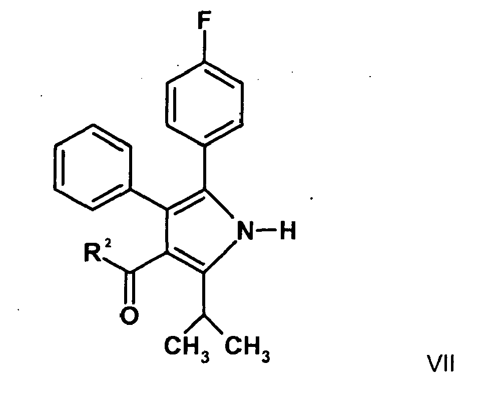 wherein R² is -OR³, -NR⁴R⁵, -NR¹⁰CONR¹¹R¹², -NR¹³OR¹⁴, -ONR¹⁵R¹⁶ or halogen, R³, R⁴, R⁵, R⁶, R⁷, R⁸, R⁹, R¹⁰, R¹¹, R¹², R¹³, R¹⁴, R¹⁵ and R¹⁶ are independently selected from hydrogen or a straight, branched and/or cyclic, saturated or unsaturated C₁₋₁₀ alkyl residue or aryl residue, both residues being optionally substituted with 1-3 optionally protected hydroxy or carboxy groups, 1-3 -OR⁷ residues, 1-3 -NR⁸R⁹ residues and/or 1-3 halogen atoms, the C₁₋₁₀ alkyl residue optionally comprising 1-3 oxygen atoms, 1-3 nitrogen atoms and/or 1-3 -NR⁶- residues, the C₁₋₁₀ alkyl residue further optionally comprising or being substituted with 1 or 2 aryl residues, to obtain a compound of the formula VIII 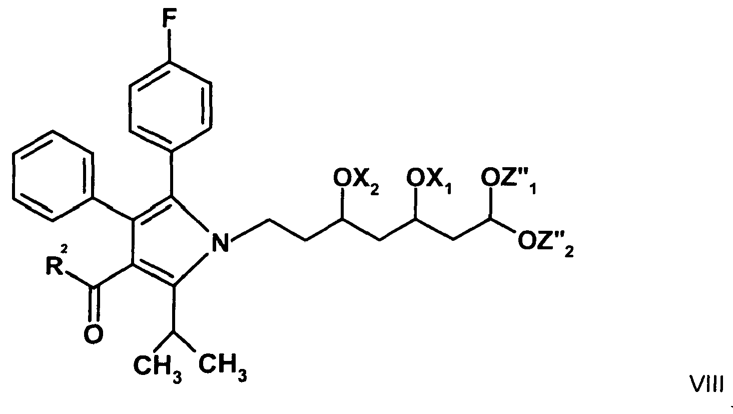 wherein R², X₁, X₂, Z"₁ and Z"₂ are defined as above.

One advantage of the process of the present invention is that if R² is -OR³ or halogen the intermediate compound of the formula VII provides the possibility of easy substitution of the R² residue, thereby providing higher flexibility for example for screening substituted pyrrole compounds for their pharmaceutical activity.

For R¹, R³, R⁴, R⁵, R⁶, R⁷, R⁸, R⁹, R¹⁰, R¹¹, R¹², R¹³, R¹⁴, R¹⁵ and R¹⁶ the alkyl residue is preferably a straight or branched, saturated or unsaturated C₁₋₆ alkyl residue or a cyclic C₃₋₆ alkyl residue, such as methyl, ethyl, n-propyl, iso-propyl, n-butyl, iso-butyl, tert-butyl, pentyl, n-hexyl or cyclohexyl. Preferred aryl residues are phenyl and naphthyl, particularly preferred phenyl. The aryl residue furthermore includes heteroaryl residues such as pyrrole or pyridine.

R² preferably is -OR³ or halogen, in particular -OR³.

In the present application halogen stands for fluoro, chloro, bromo or iodo, preferably chloro or bromo.

The compound of the formula VII can be obtained from reacting a compound of the formula XVII 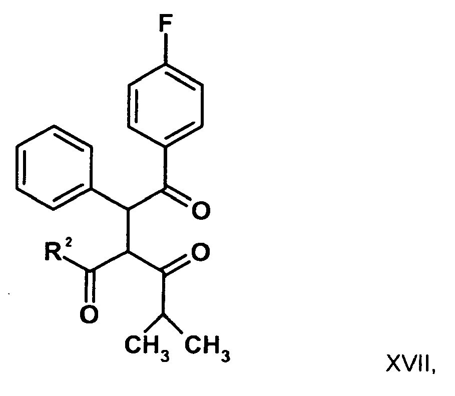 wherein R² is defined as above, or a salt thereof with a compound of the formula R¹-NH₂, wherein R¹ is preferably a hydrogen.

If in the compound of formula R¹-NH₂ R¹ is hydrogen, liquid ammonia, aqueous ammonia or a solution of an ammonium salt such as NH₄Cl or CH₃COONH₄ can be used. Thus, NH₃ as one compound of the formula R¹-NH₂ also includes NH₄⁺ ions which will always be present in the reaction solution in dependence of the pH value of the solution. Preferably the diketone of the formula XVII is reacted with CH₃COONH₄. The reaction can be carried out in any common inert solvent such as THF preferably at elevated temperatures, such as, for example, under reflux.

Moreover, if using NH₃ as the compound of the formula R¹-N₂ in the process of the present invention, the yield of the ring-forming reaction to obtain the compound of formula VII is significantly higher compared to reactions with compounds of the formula R¹-NH₂, wherein R¹ is other than hydrogen.

Preferably the reaction is conducted with ammonium acetate in refluxing THF for about 6 hours. The yield of this reaction is considerably higher than the yield of the similar reaction with benzylamine instead of ammonium acetate.

Further detailed reaction conditions can for example be found in WO 2004/046105, WO 94/20492 and EP-A-0 330 172. For example, the reaction can be conducted under heating with azeotropical water removed in an appropriate solvent or mixture of solvents catalyzed with an acid, such as, for example, pivalic acid. As suitable solvents n-heptane, toluene and THF or mixtures thereof can be mentioned. Alternatively the reaction can be conducted without any solvent (neat) under heating the reactants to for example about 120°C to 140°C.

The compound of the formula XVII can be obtained by
a) reacting a compound of the formula XXV 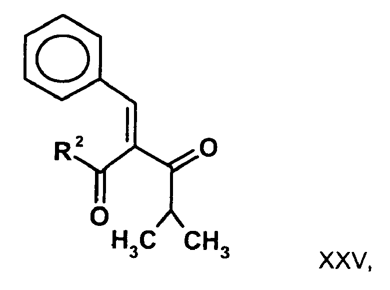
   wherein R² is defined as above,
   with 4-fluorobenzaldehyde or
b) reacting a compound of the formula XXVI 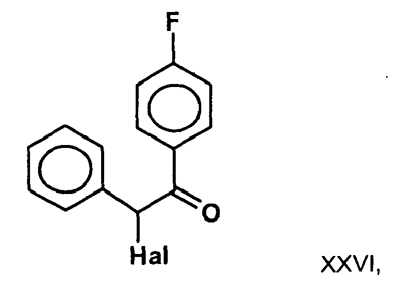 wherein Hal is halogen, preferably bromo with a compound of the formula XXVII 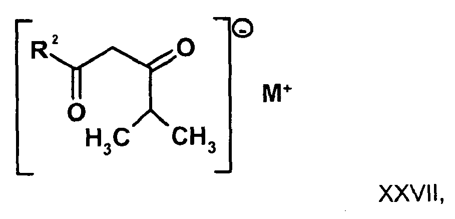 wherein R² is defined as above and M⁺ is selected from H⁺, Li⁺, Na⁺ and K⁺, preferably Na⁺.

The above reaction route a) has the advantage that the compound of the formula XXV wherein R² is -OR³ can be easily purified by distillation. Thus, this intermediate can be employed in the following reaction in a highly pure form.

The above reaction route b) has the advantage that the compound of the formula XXVI can easily be obtained from the commercially available 2-[2-(4-fluorophenyl)-2-oxo-1-phenylethyl]-4-methyl-3-oxo-pentanoic acid phenylamide by cleavage with hydrogen peroxide in the presence of a base such as NaOH.

Above reaction a) can for example be carried out under solvent-free conditions catalyzed by 2-(2-hydroxyethyl)-3-methyl-4-ethylthiazolium bromide in the presence of triethylamine under heating to for example to a temperature of about 60°C to about 80°C.

In reaction route a) the compound of the formula XXV can be obtained by reacting a compound of the formula XXVIII 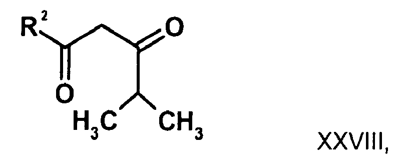 wherein R² is defined as above with benzaldehyde. This reaction can be carried out in the presence of a catalyst such as, for example, piperidine and glacial acetic acid, ethylene diamine and glacial acetic acid, β-alanine and glacial acetic acid, and the like in an inert solvent such as, for example, toluene, heptane, hexane, and the like for about 24 to about 36 hours at about 60°C to about 120°C with the removal of water to afford a compound of the formula XXV.

If in the compound of the formula XXV R² is -OR³, this compound is heat-stable and can be distilled as to separate the desired compound of the formula XXV from undesired byproducts of the reaction. In a preferred embodiment the reaction between the compound of the formula XXVIII and benzaldehyde is catalyzed with β-alanine in the presence of acetic acid, preferably glacial acetic acid, in toluene. The reaction mixture is heated under reflux with azeotropic removal of water for about 24 hours. After usual workup the product can be distilled under reduced pressure.

In the alternative embodiment according to above route b) the compound of the formula XVII is obtained by reacting the compounds of the formula XXVI and XXVII with each other. This addition reaction can be carried out in any suitable inert organic solvent, preferably anhydrous inert organic solvent, such as, for example, ethers, e.g. diethyl ether, 1,2-diethoxyethane, 1,2-dimethoxyethane, THF or mixtures thereof. Preferably the reaction is carried out in THF. The reaction temperature may be in the range of for example about 0°C to about 40°C, preferably from about 0°C to about room temperature.

In a preferred embodiment the compound of the formula XXVII is prepared in situ.

The compound of the formula XXVI can be obtained by halogenating the compound of the formula XXIX

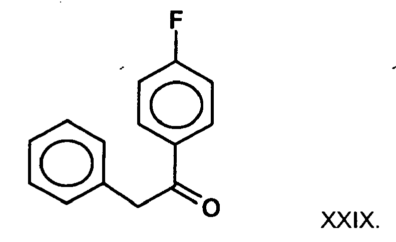

Halogenation, preferably bromination of the compound of the formula XXIX can be carried out with for example bromine in an inert organic solvent, preferably an anhydrous inert organic solvent, such as, for example, halogenated lower alkane solvents, e.g. CCl₄, CHCl₃, 1,1-dichloroethane, 1,2-dichloroethane, methylene chloride, 1,1,2-trichloroethane or mixtures thereof. A preferred solvent is CHCl₃. The reaction can be carried out for example at a temperature of about 0°C to about 40°C.

The compound of the formula XXIX can be obtained by cleavage of, for example, the commercially available 2-[2-(4-fluorophenyl)-2-oxo-1-phenyl-ethyl]-4-methyl-3-oxo-pentanoic acid phenylamide. The cleavage can be carried out for example with hydrogen peroxide in the presence of a base such as NaOH.

A particularly advantageous route for the synthesis of a compound of the formula XVII is shown in the following scheme 4:

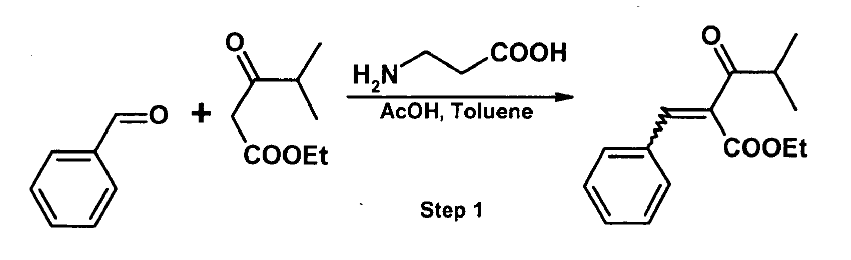

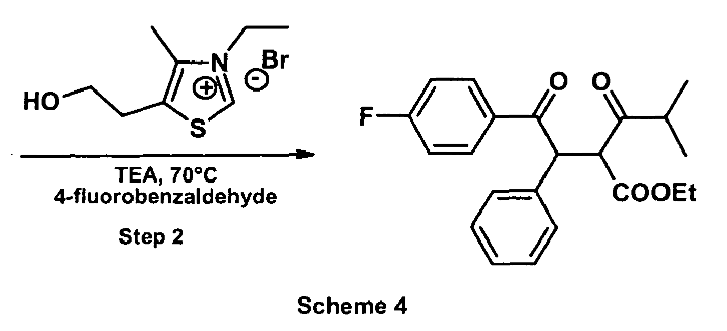

Advantages of the synthesis of diketone ethyl ester by this route are:
- no need of reaction solvent in step 2 (environmental, economic);
- no need of recrystallization process in step 1 (environmental, economic), product is isolated by distillation;
- this is the shortest synthetic route (only 2 steps) in comparison with other known processes (3-5 steps required);
- in the process for the step 1 nontoxic catalyst (β-alanine) is used (environmental);
- no need of toxic a corrosive reagents (like bromine in alternative route);
- all starting materials are commercially available.

The obtained pyrrole derivatives of the formula VIII are useful intermediates in the preparation of atorvastatin.

In one embodiment the process of the present invention comprises the step of reacting the compound of VIb, wherein W is NH₂, with a compound of the formula XVII 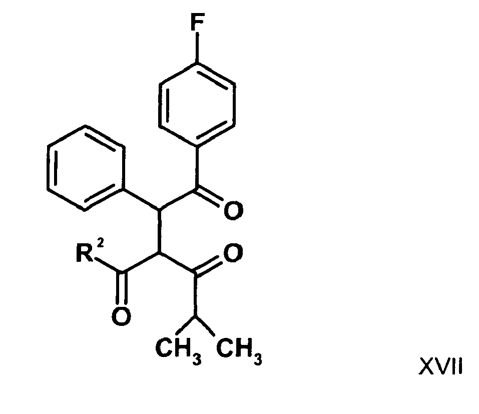 wherein R₂ is defined as above to obtain a compound of the formula VIII 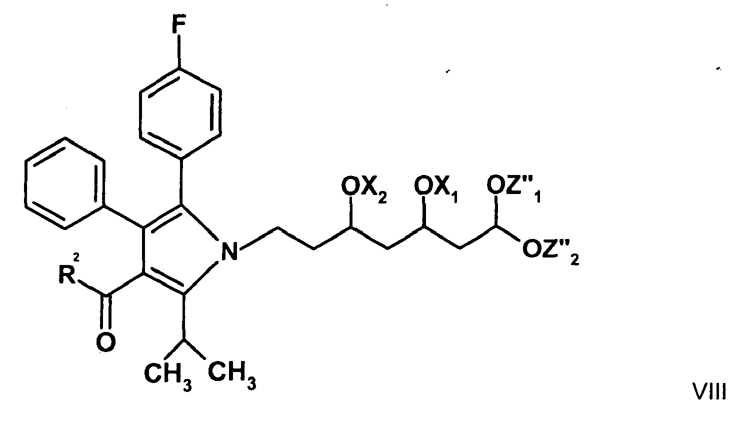 wherein R², X₁, X₂, Z"₁ and Z"₂ are defined as above.

As the next step, the process of the present invention preferably further comprises the step of converting and optionally deprotecting and/or protecting any protected or unprotected group of a compound of the formula VIII to obtain a compound of the formula IX 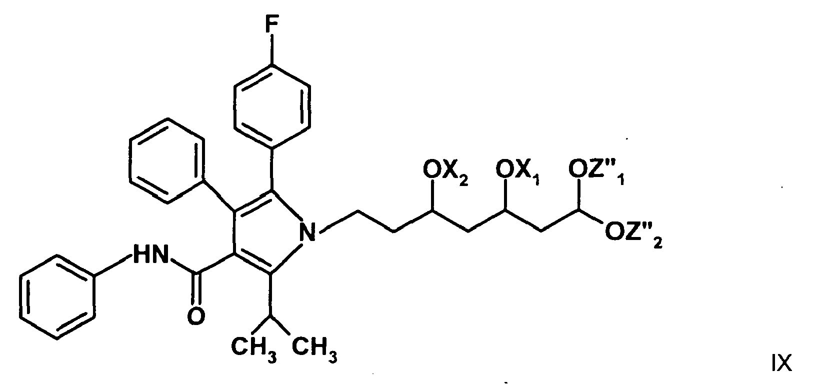 wherein X₁, X₂, Z"₁ and Z"₂ are defined as above.

As another step, the process of the present invention preferably further comprises the step of hydrating and optionally deprotecting and/or protecting any protected or unprotected group of a compound of the formula IX to obtain a compound of the formula X 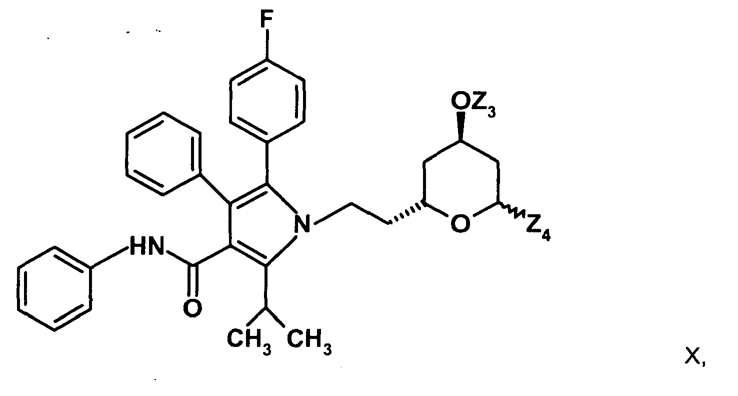 wherein Z₃ is H or a hydroxy protecting group, Z₄ is =O, OH or OZ₅ and Z₅ is a hydroxy protecting group.

Methods for modification of protecting groups are known to the person skilled in the art as exemplified in the examples.

In the process of the present invention the compound of the formula I has preferably the formula I' 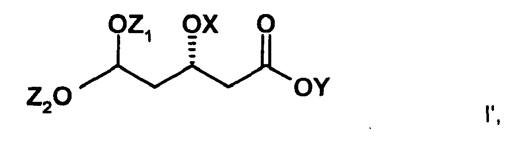 wherein Z₁, Z₂, X and Y are defined as above.

The present invention further relates to a process for preparing a compound of the formula VI 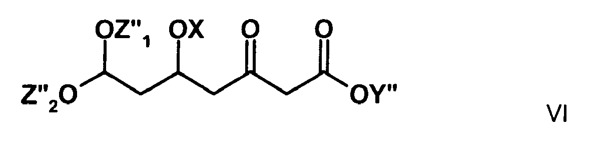 or a salt thereof, wherein Z"₁ is a hydroxy protecting group, Z"₂ is a hydroxy protecting group, X is H or a hydroxy protecting group and Y" is a carboxy protecting group, which process comprises the step of reacting the compound of formula I 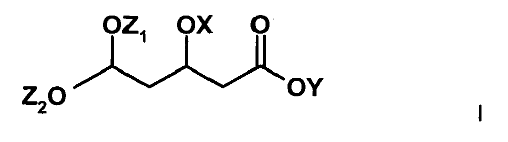 or a salt thereof, wherein Z₁ is a hydroxy protecting group, Z₂ is a hydroxy protecting group, X is H or a hydroxy protecting group, and Y is H or a carboxy protecting group, with a compound of formula XV 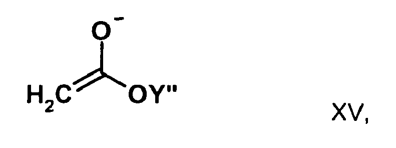 wherein Y" is a carboxy protecting group, and optionally deprotecting and/or protecting any protected or unprotected group.

The present invention further relates to a process for preparing a compound of the formula VIa 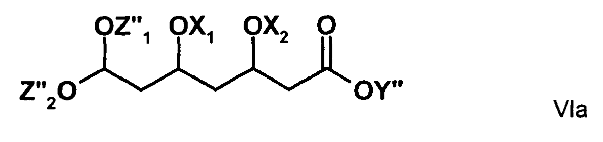 or a salt thereof, wherein Z"₁ is a hydroxy protecting group, Z"₂ is a hydroxy protecting group, X₁ and X₂ can be independent from each other a hydroxy protecting group or X₁ and X₂ taken together may form a ring with the oxygen and intermediate carbon atoms to which they are attached and Y" is a carboxy protecting group, which process comprises the step of achiral or chiral enantioselective hydrogenation of a compound of formula VI 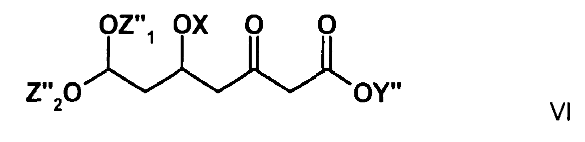 or a salt thereof, wherein Z"₁ is a hydroxy protecting group, Z"₂ is a hydroxy protecting group, X is H or a hydroxy protecting group and Y" is defined as above and optionally deprotecting and/or protecting any protected or unprotected group.

The present invention further relates to a process for preparing a compound of the formula VIb 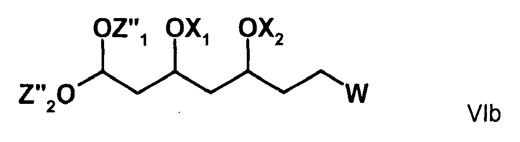 wherein Z"₁ is a hydroxyl protecting group, Z"₂ is a hydroxyl protecting group, X₁ and X₂ can be independent from each other a hydroxyl protecting group or X₁ and X₂ taken together may form a ring with the oxygen and intermediate carbon atoms to which they are attached and W is NH₂, N₃, OH, OW₁ or a leaving group and W₁ is a hydroxyl protecting group, which process comprises the step of hydrogenation and/or group modification of a compound of formula VIa 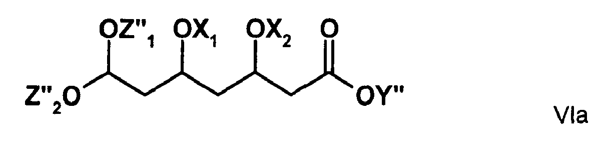 or a salt thereof, wherein Z"₁ is a hydroxy protecting group, Z"₂ is a hydroxy protecting group, X₁ and X₂ can be independent from each other a hydroxy protecting group or X₁ and X₂ taken together may form a ring with the oxygen and intermediate carbon atoms to which they are attached and Y" is a carboxy protecting group.

The present invention further relates to a process for preparing a compound of the formula VIII 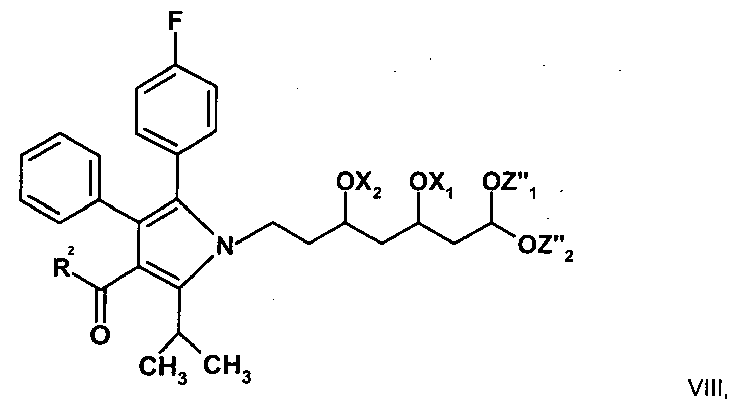 wherein R² is -OR³, -NR⁴R⁵, -NR¹⁰CONR¹¹R¹², -NR¹³OR¹⁴, -ONR¹⁵R¹⁶ or halogen, R³, R⁴, R⁵, R⁶, R⁷, R⁸, R⁹, R¹⁰, R¹¹, R¹², R¹³, R¹⁴, R¹⁵ and R¹⁶ are independently selected from hydrogen or a straight, branched and/or cyclic, saturated or unsaturated C₁₋₁₀ alkyl residue or aryl residue, both residues being optionally substituted with 1-3 optionally protected hydroxy or carboxy groups, 1-3 -OR⁷ residues, 1-3 -NR⁸R⁹ residues and/or 1-3 halogen atoms, the C₁₋₁₀ alkyl residue optionally comprising 1-3 oxygen atoms, 1-3 nitrogen atoms and/or 1-3 -NR⁶ residues, the C₁₋₁₀ alkyl residue further optionally comprising or being substituted with 1 or 2 aryl residues, and X₁, X₂, Z"₁ and Z"₂ are defined as above, which process comprises reacting a compound of the formula VII 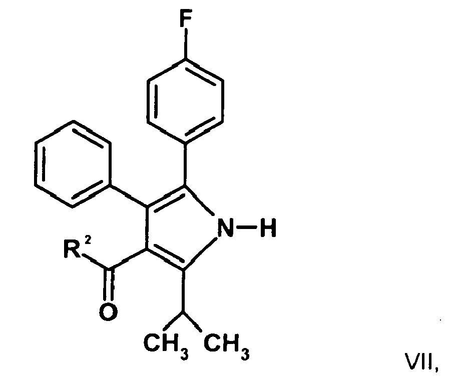 wherein R² is defined as above, with a compound of the formula VIb 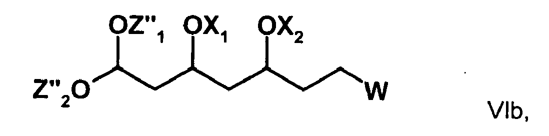 wherein W is a leaving group and X₁, X₂, Z"₁ and Z"₂ are defined as above.

The present invention further relates to a process for preparing a compound of the formula VIII 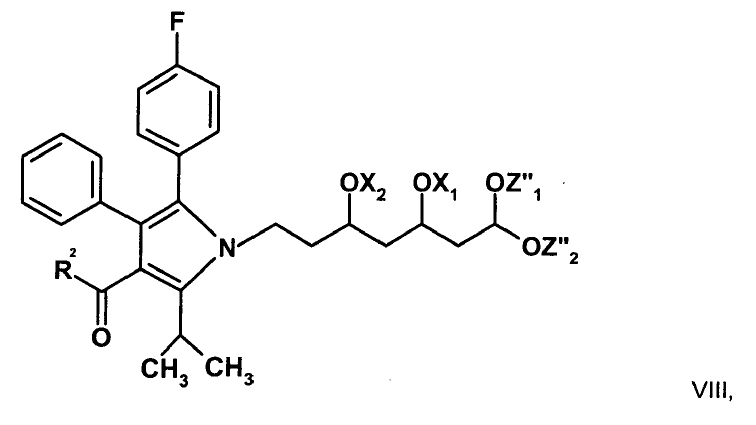 wherein R² is -OR³, -NR⁴R⁵, -NR¹⁰CONR¹¹R¹², -NR¹³OR¹⁴, -ONR¹⁵R¹⁶ or halogen, R³, R⁴, R⁵, R⁶, R⁷, R⁸, R⁹, R¹⁰, R¹¹, R¹², R¹³, R¹⁴, R¹⁵ and R¹⁶ are independently selected from hydrogen or a straight, branched and/or cyclic, saturated or unsaturated C₁₋₁₀ alkyl residue or aryl residue, both residues being optionally substituted with 1-3 optionally protected hydroxy or carboxy groups, 1-3 -OR⁷ residues, 1-3 -NR⁸R⁹ residues and/or 1-3 halogen atoms, the C₁₋₁₀ alkyl residue optionally comprising 1-3 oxygen atoms, 1-3 nitrogen atoms and/or 1-3 -NR⁶ residues, the C₁₋₁₀ alkyl residue further optionally comprising or being substituted with 1 or 2 aryl residues, and X₁, X₂, Z"₁ and Z"₂ are defined as above, which process comprises reacting a compound of the formula XVII 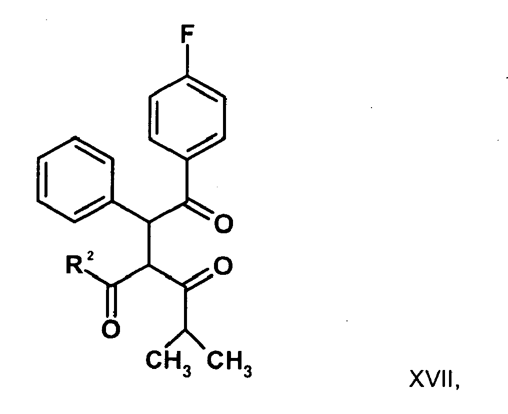 wherein R² is defined as above, with a compound of the formula VIb 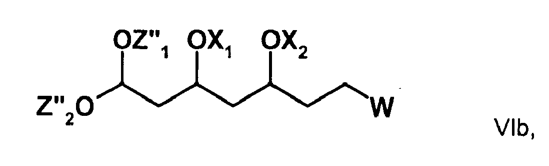 wherein W is NH₂ and X₁, X₂, Z"₁ and Z"₂ are defined as above.

The present invention further relates to a process for preparing a compound of the formula IX 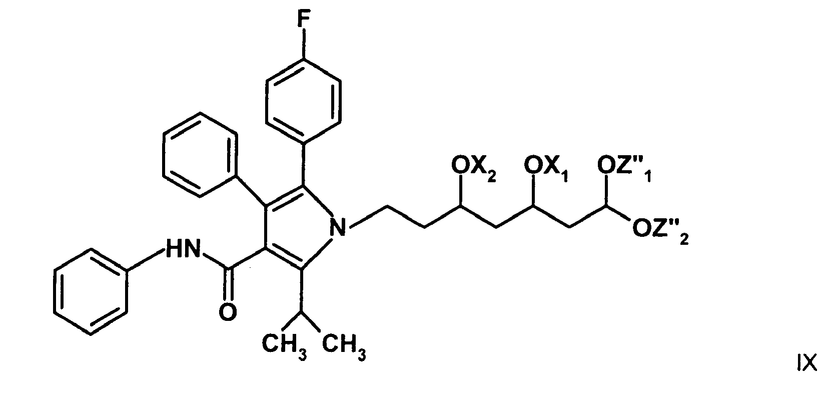 wherein X₁, X₂, Z"₁ and Z"₂ are defined as above, which process comprises the step of conversion of a compound of the formula VIII 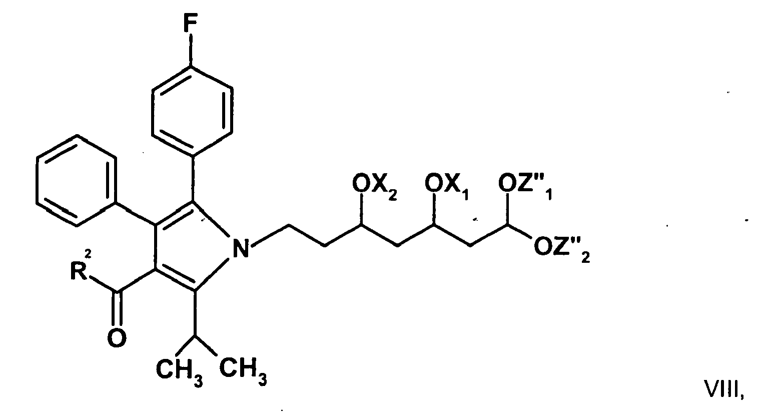 wherein R² is -OR³, -NR⁴R⁵, -NR¹⁰CONR¹¹R¹², -NR¹³OR¹⁴, -ONR¹⁵R¹⁶ or halogen, R³, R⁴, R⁵, R⁶, R⁷, R⁸, R⁹, R¹⁰, R¹¹, R¹², R¹³, R¹⁴, R¹⁵ and R¹⁶ are independently selected from hydrogen or a straight, branched and/or cyclic, staturated or unsaturated C₁₋₁₀ alkyl residue or ary residue, both residues being optionally substituted with 1-3 optionally protected hydroxy or carboxy groups, 1-3 -OR⁷ residues, 1-3 -NR⁸R⁹ residues and/or 1-3 halogen atoms, the C₁₋₁₀ alkyl residue optionally comprising 1-3 oxygen atoms, 1-3 nitrogen atoms and/or 1-3 -NR⁶ residues, the C₁₋₁₀ alkyl residue further optionally comprising or being substituted with 1 or 2 aryl residues, and X₁, X₂, Z"₁ and Z"₂ are defined as above.

The present invention further relates to a process for preparing a compound of the formula X 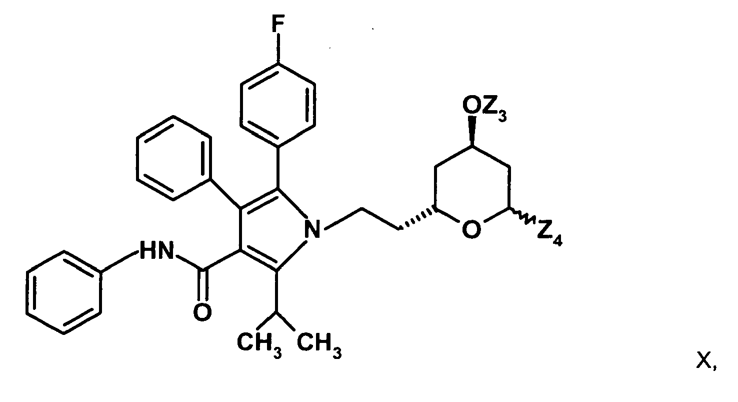 wherein Z₁ is H or a hydroxy protecting group, Z₂ is =O, OH or OZ₃ and Z₃ is a hydroxy protecting group, which process comprises the step of hydration of the compound of formula IX 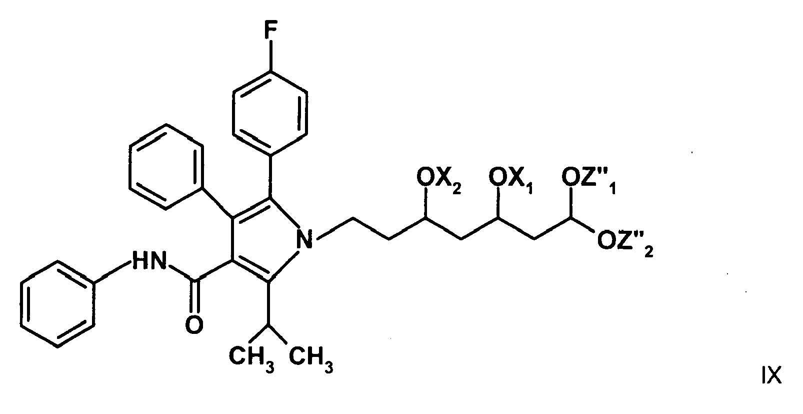 wherein X₁, X₂, Z"₁ and Z"₂ are defined as above, and optionally deprotecting and/or protecting any protected or unprotected group.

The present invention further relates to the compounds of the formula XI 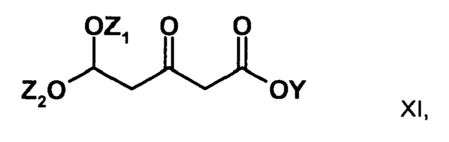 wherein Z₁ is hydroxyl protecting group, Z₂ is a hydroxyl protecting group and Y is a carboxyl protecting group;
of the formula XIa 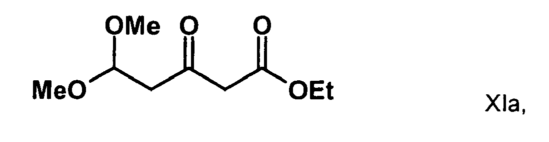 of the formula XIIa and XIIb 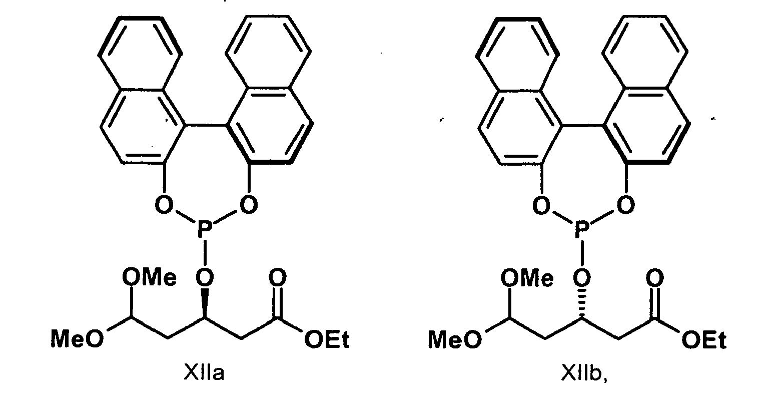 of the formula VIII 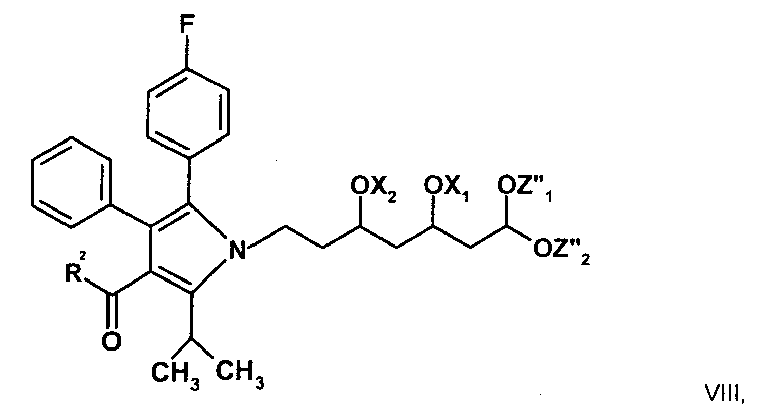 of the formula XIII 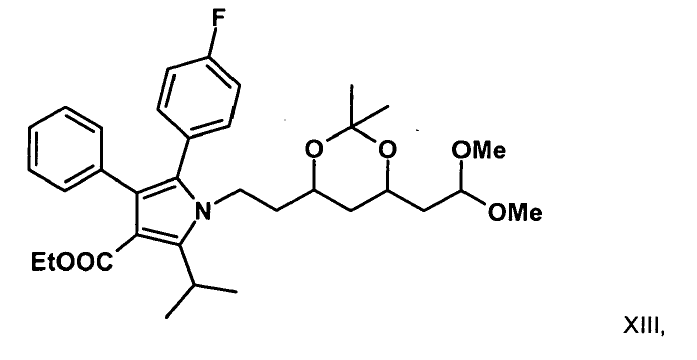 of the formula XIV 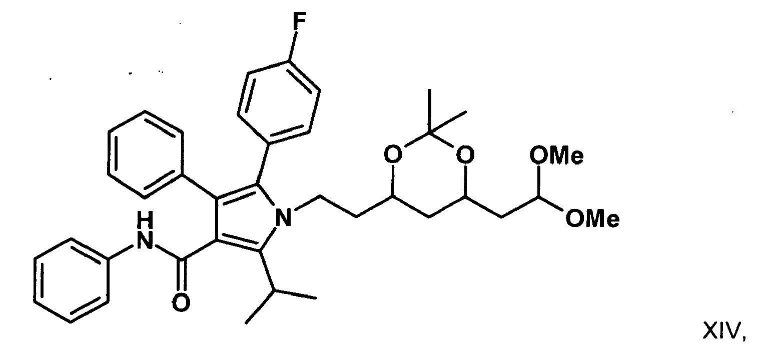 of the formula XVI 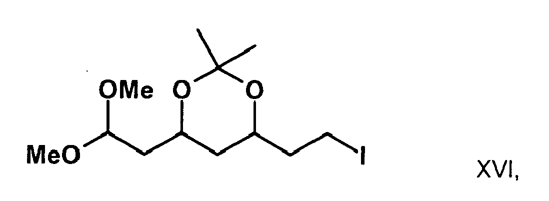 of the formula XVIII 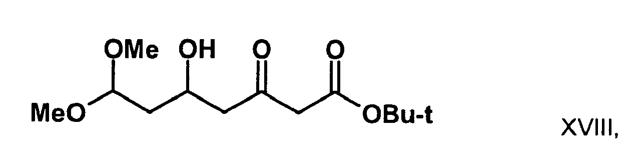 of the formula XIX 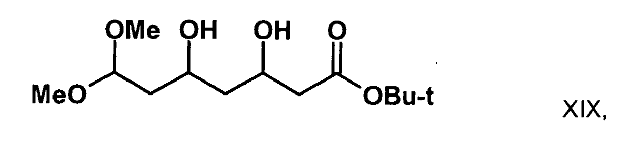 of the formula XX 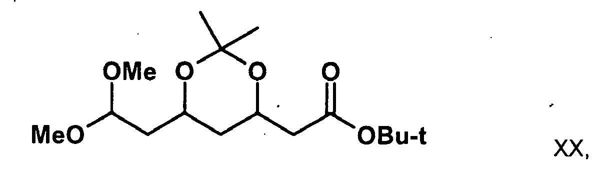 of the formula XXI 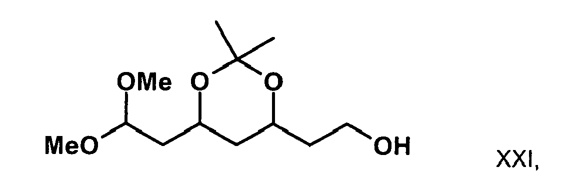 of the formula XXII 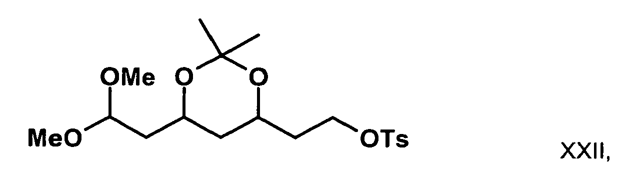 of the formula XXIII 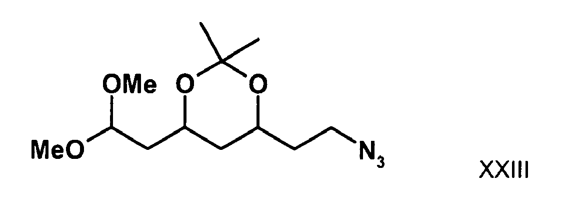 and of the formula XXIV

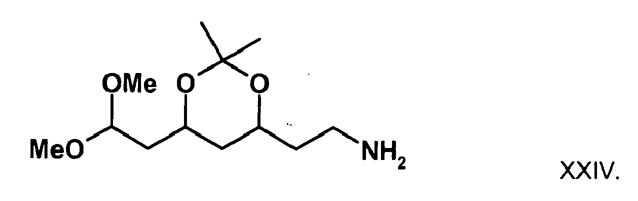

The present invention further relates to the compounds of formulae IV, V, VI, VIa, VIb and IX.

These compounds are useful intermediates in the preparation of atorvastatin.

Within this application, all educts, intermediates and products to be used in the processes of the present invention may be used as racemates or enantiomerially enriched mixtures, e.g. mixtures which are enriched in one enantiomer or comprise only one substantially purified enantiomer.

Each process of the present invention can further comprise one or more steps of separation or enrichment of enantiomers, e.g. steps of racemic separation. Methods of separation or enrichment of enantiomers are known in the art.

Preferably the stereo configuration of educts, intermediates and products is chosen such that when used in processes of the present invention the intermediates and products resulting from said processes show the stereo configuration suitable for the preparation of atorvastatin or are in or correspond to the stereo configuration of atorvastatin.

The present invention will now be further illustrated by the following examples which are not intended to be limiting.

### Example 1: Ethyl 5,5-dimethoxy-3-oxo-pentanoate (3)

Solid N,N'-carbonyldiimidazole (Im₂CO) (50.02 g, 0.308 mol) was added in portions during 5-10 min to a solution of acid **2** (41.45 g, 0.288 mol) in THF (200 ml) (gas evolution). Then the mixture was stirred at RT for 2 h to produce *in situ* the corresponding imidazolide. In another flask, a mixture of monoethyl malonate (83.5 g, 0.632 mol) and commercial Mg powder with particle size <0.1 mm (10.24 g, 0.422 mol) in THF (300 ml) was stirred under reflux for 4 h to produce *in situ* Mg(OOCCH₂COOEt)₂, which was cooled down to RT and added to the former solution. The residual Mg powder was rinsed with THF (100 ml). The wash solution was added to the reaction mixture. The mixture was stirred overnight at RT and concentrated in vacuum. The residue was dissolved in ethyl acetate (300 ml) and acidified with 2M NaHSO₄ (620 ml) under vigorous stirring. The organic layer was separated, washed successively with water, sat. NaHCO₃ (3x200 ml) and brine. It was subsequently dried over Na₂SO₄ and evaporated. Vacuum distillation of the residue (b.p. 86-92 °C/0.06 mbar) afforded 53.62 g (91 % yield) of keto ester **3**. ¹H-NMR (CDCl₃): 1.28 (t, J 7.1 Hz, 3H), 2.86 (d, J 5.6Hz, 2H), 3.37 (s, 6H), 3.49 (s, 2H), 4.20 (q, *J* 7.1Hz, 2H), 4.78 (t, J 5.6Hz, 1 H).

### Example 2: Ethyl 5,5-dimethoxy-3-hydroxy-pentanoate (4) (compound of formula I)

### Catalytic hydrogenation of 3

Keto ester **3** (2.04 g, 10 mmol) and Ru((*R*)-BINAP)Cl₂ (0.004 g, 0.005 mmol) were placed in an autoclave under argon followed by addition of abs. MeOH (8 ml). The mixture was pressurized with hydrogen to 50 bar and stirred at 50 °C until the H₂ consumption stopped (1 h). Evaporation of the solvent in vacuum gave hydroxy ester **4** as yellowish oil in quantitative yield. ¹H-NMR (CDCl₃): 1.27 (t, *J* 7.1 Hz, 3H), 1.75-1.83 (m, 2H), 2.46-2.51 (m, 2H), 3.22 (br., 1H), 3.37 (s, 6H), 4.17 (q, *J* 7.1 Hz, 2H), 4.15-4.23 (m, 1H), 4.61 (t, *J* 5.7Hz, 1 H). ¹³C-NMR (CDCl₃): 14.6, 39.4, 42.0, 53.6, 53.9, 61.0, 65.3, 103.3, 172.6.

### Determination of the enantiomeric composition of compound 4 (according to scheme 3)

A 10% solution of (*S*)-XII ((0.5 ml, 0.14 mmol) in dry toluene, the alcohol **4** (21 mg, 0.10 mmol), abs. Et₃N ((20 mg, 0.19 mmol) and dry d₆-benzene (0.1 ml) were mixed together in a vial. The resultant pale solution was immediately transferred into an NMR-tube under argon and then the ³¹P NMR spectrum was recorded. Two singlet peaks at δ_{P} 151.4 and 154.6 separated till the baseline correspond to compounds **XIIa** and **XIIb**, respectively. The enantiomeric excess of the alcohol **4** was calculated from the integral intensities of the peaks.

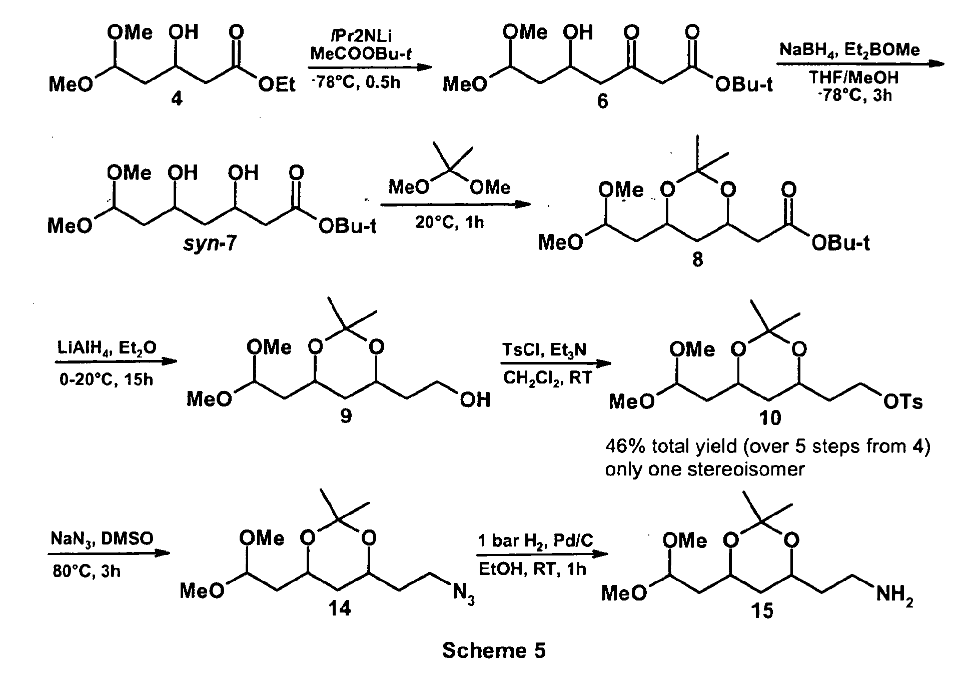

### Example 3: Tertbutyl-7,7-dimethoxy-5-hydroxy-3-oxo-heptanoate (6) (compound of formula VI)

*Tert*-butyl acetate (43.36 g, 0.37 mol) was added neat to 2.0 M solution of LDA in THF/heptane (175 ml, 0.35 mol) at -78°C. After the solution was stirred for 15 min, hydroxy ester **4** (18.37 g, 0.089 mol) was added neat. The reaction mixture was warmed to RT during 1 h, quenched with saturated NH₄Cl solution (200 ml) and stirred for 1 h. The mixture was extracted twice with ethyl acetate; combined organic layers were washed with brine twice and dried over Na₂SO₄. Evaporation of the solvent in vacuum gave crude compound 6 as brown viscous oil (33.19 g), which was used further without additional purification. Analytically pure sample was obtained by column chromatography (silica, hexane/EtOAc = 1/1, R_{f} 0.24). ¹H-NMR (CDCl₃): 1.47 (s, 9H), 1.73-1.82 (m, 2H), 2.69-2.76 (m, 2H), 3.25-3.36 (m, 1H), 3.36 (s, 3H), 3.37 (s, 3H), 3.39 (s, 2H), 4.25 (br, 1 H), 4.59 (t, *J* = 5.5 Hz, 1 H). ¹³C-NMR (CDCl₃): 28.0, 39.0, 49.6, 51.2, 53.4, 53.7, 64.5, 82.2, 103.2, 166.2, 203.3. Found: C 56.70, H 9.02; calcd. for C₁₃H₂₄O₆: C 56.51, H 8.75.

### Example 4: Tertbutyl-3,5-dihydroxy-7,7-dimethoxy-heptanoate (7) (compound of formula VIa)

24.87 g (0.09 mol) of crude **6** was dissolved in a mixture of abs. THF (300 ml) and abs. MeOH (60 ml), the solution was cooled with dry ice/acetone bath and 100 ml of 1.0M solution of Et₂BOMe in THF was added via cannula. In 1 h time, NaBH₄ (6.43 g, 0.17 mol) was added in portions. The obtained pale yellow solution was stirred at -78°C for 2.5 h, then warmed to 0°C and quenched with water solution of NaHSO₄ (21.6 g, 0.18 mol). 100 ml of methanol and 40 ml of 30% H₂O₂ were added at 0°C, the mixture was stirred for 1 h at RT and then partioned between ethyl acetate and brine. Organic layer was separated and the water layer extracted one more time with ethyl acetate. Combined organic extracts were washed with water, brine and water solution of 20 g Na₂SO₃ (heat evolves!), then dried with Na₂SO₄ and concentrated in vacuum to obtain crude compound **7** as yellow-brown oil (21.9 g). It was used further without additional purification. For analytic purposes, the compound was purified by column chromatography (silica, EtOAc, R_{f} 0.48). ¹H-NMR (CDCl₃): 1.46 (s, 9H), 1.56-1.65 (m, 2H), 1.72-1.81 (m, 2H), 2.38-2.44 (m, 2H), 3.36 (s, 6H), 3.92 (br, 1 H), 4.05 (m, 2H), 4.20-4.28 (m, 1 H), 4.59 (t, *J* = 5.7 Hz, 1 H). ¹³C-NMR (CDCl₃): 28.0, 40.0, 42.6, 42.8, 53.2, 53.5, 68.3, 68.4, 81.1, 103.2, 171.7. Found: C 56.10, H 10.11; calcd. for C₁₃H₂₆O₆: C 56.10, H 9.42.

### Example 5: (8) (compound of formula VIa)

19.8 g (0.071 mol) of crude **7** and 1.35 g of p-toluenesulfonic acid monohydrate (0.071 mol) were dissolved in 50 ml of 2,2-dimethoxypropane and the resulting solution was stirred at RT overnight. Solid NaHCO₃ (4.7 g, 0.056 mol) was added followed by 50 ml of water and the solution was stirred for 1 h. The mixture was extracted with ethyl acetate twice, combined organic extracts washed with water and brine, dried (Na₂SO₄) and concentrated in vacuum to obtain crude **8** as brown oil (20.65 g), which was used further without additional purification. For analytic purposes, the compound was purified by column chromatography (silica, hexane/EtOAc = 2/1). ¹H-NMR (CDCl₃): 1.14-1.28 (m, 1H), 1.36 (s, 3H), 1.44 (s, 9H), 1.45 (s, 3H), 1.53-1.60 (m, 1H), 1.68-1.79 (m, 2H), 2.25-2.47 (m, 2H), 3.32 (s, 3H), 3.35 (s, 3H), 3.94-4.04 (m, 1 H), 4.20-4.31 (m, 1 H), 4.52-4.58 (m, 1 H). ¹³C-NMR (CDCl₃): 19.7, 28.1, 30.1, 36.5, 39.6, 42.7, 53.0, 53.6, 65.6, 66.2, 80.6, 98.7, 101.6, 170.2. Found: C 60.14, H 10.00; calcd. for C₁₆H₃₀O₆: C 60.35, H 9.50. MS (El, 70 eV): 303 ([M - 15]⁺, 23%), 245 ([M - 73]⁺, 9%), 155 ([M - 163]⁺, 37%), 75 (100%).

### Example 6: (9) (compound of formula VIb)

LiAlH₄ (2.35 g, 0.062 mol) was added in portions to a solution of crude **8** (19.7 g, 0.062 mol) in 300 ml of dry ether at 0°C, and the resulting mixture was left stirring at RT overnight. 13.4 ml of 2N NaOH was added at 0°C, the resulting solution was stirred at RT for 5 h, filtered and the filter cake washed with ether. The combined filtrates were washed with brine twice, dried (Na₂SO₄) and concentrated in vacuum to obtain red-brown oil of crude **9** (12.93 g), which was used further without additional purification. Analytically pure sample was obtained by column chromatography (silica, EtOAc). ¹H-NMR (C₆D₆): 1.22-1.38 (m, 2H), 1.48 (s, 3H), 1.60-1.68 (m, 1 H), 1.61 (s, 3H),1.80-1.89 (m, 1 H), 1.90-2.09 (m, 2H), 2.61 (br, 1 H), 3.33 (s, 3H), 3.38 (s, 3H), 3.78-3.95 (m, 2H), 3.96-4.04 (m, 1H), 4.07-4.15 (m, 1H), 4.82-4.87 (m, 1H). ¹³C-NMR (C₆D₆): 20.1, 30.7, 37.5, 39.3, 40.6, 52.7, 53.4, 60.2, 66.3, 68.6, 98.8, 102.1. Found: C 58.14, H 10.19; calcd. for C₁₂H₂₄O₅: C 58.04, H 9.74;

### Example 7: (10) (compound of formula VIb)

5.72 g (0.03 mol) of *p*-toluenesulfonyl chloride was added to a stirred solution of crude **9** (4.97 g, 0.02 mmol) and triethylamine (6.95 ml, 0.05 mol) in 60 ml of dry CH₂Cl₂ at 0°C. The resulting solution was stirred at RT overnight and quenched with 50 ml of water. Organic layer was separated and the water layer extracted with dichloromethane. Combined organic extracts were washed twice with brine, dried (Na₂SO₄) and concentrated in vacuum to obtain dark brown viscous oil. Purification with column chromatography (silica, hexane/EtOAc = 2/1) afforded 4.56 g of **10** as brownish oil. ¹H-NMR (C₆D₆): 0.97-1.04 (m, 2H), 1.33 (s, 3H), 1.46 (s, 3H), 1.54-1.61 (m, 2H), 1.74-1.81 (m, 1H), 1.86-1.94 (m, 1H), 1.97 (s, 3H), 3.23 (s, 3H), 3.29 (s, 3H), 3.69-3.76 (m, 1 H), 3.87-4.07 (m, 2H), 4.19-4.26 (m, 1H), 4.71-4.75 (m, 1H), 6.84 (d, J = 8.1 Hz, 2H), 7.85 (d, J = 8.1Hz, 2H). ¹³C-NMR (C₆D₆): 20.0, 21.4, 30.6, 36.1, 37.3, 40.4, 52.7, 53.4, 65.3, 66.0, 67.1, 98.9, 102.0, 128.4, 130.1, 134.6, 144.5.

### Example 8: (14) (compound of formula VIb)

A solution of **10** (1.24 g, 3.1 mmol) and NaN₃ (0.358 g, 5.5 mmol) in 6 ml of dry DMSO was stirred at 80°C for 3h, then 10 ml of water was added and the solution was extracted with ethyl acetate 3 times. Combined organic extracts were washed with brine, dried (Na₂SO₄) and concentrated in vacuum to obtain crude **14** as brownish oil (0.78 g, 93% yield), which was used further without additional purification. Analytically pure sample was obtained by column chromatography (silica, hexane/EtOAc = 2/1). ¹H-NMR (C₆D₆): 1.12-1.20 (m, 2H), 1.43-1.52 (m, 1H), 1.51 (s, 3H), 1.53-1.65 (m, 1H), 1.68 (s, 3H), 1.91-2.12 (m, 2H), 3.05-3.13 (m, 1 H), 3.22-3.29 (m, 1 H), 3.36 (s, 3H), 3.42 (s, 3H), 3.76-3.84 (m, 1 H), 4.06-4.14 (m, 1H), 4.86-4.90 (m, 1H). ¹³C-NMR (C₆D₆): 20.0, 30.7, 36.1, 37.4, 40.5, 47.8, 52.7, 53.4, 66.1, 66.2, 98.9, 102.1. MS (EI, 70 eV): 258 ([M - 15]⁺, 12%). Found: C 52.30, H 8.84; calcd. for C₁₂H₂₃N₃O₄: C 52.73, H 8.48.

### Example 9: (15) (compound of formula VIb)

0.78 g (2.85 mmol) of **14** and 0.078g of 5% Pd/C were placed in a flask, which was then filled with hydrogen (3 cycles of vacuum/H₂). Abs. EtOH (12 ml) was added, the mixture stirred under hydrogen for 2 h and filtered. The filter cake was washed with ethanol and combined filtrates were concentrated in vacuum to obtain **15** as yellow oil (0.68 g, 96% yield). ¹H-NMR (C₆D₆): 0.79 (br, 1H), 1.06-1.12 (m, 2H), 1.24-1.35 (m, 1H), 1.32 (s, 3H), 1.48 (s, 3H), 1.49-1.58 (m, 1H), 1.72-1.79 (m, 1H), 1.85-1.92 (m, 1H), 2.63 (t, *J* = 6.7 Hz, 2H), 3.14 (s, 3H), 3.19 (s, 3H), 3.69-3.76 (m, 1H), 3.90-3.98 (m, 1H), 4.66-4.71 (m, 1H). ¹³C-NMR (C₆D₆): 20.0, 30.7, 37.7, 38.7, 40.3, 40.8, 52.4, 53.2, 66.1, 67.4, 98.5, 101.9.

### Example 10: (13) (compound of formula VIb)

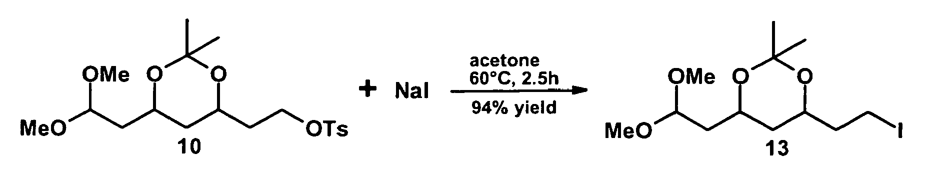

A solution of the tosylate **10** (2.01 g, 5 mmol) and dry sodium iodide (2.25 g, 15 mmol) in 30 ml of dry acetone was stirred at 60°C for 2.5 h. The solvent was then removed in vacuum and the solid residue dissolved in EtOAc/water. Organic layer was isolated, the water layer extracted with EtOAc, the combined organic extracts were washed with brine twice, dried (Na₂SO₄) and concentrated in vacuum to afford 1.94 g of dark red-brown viscous oil. Column chromatography (silica, hexane/EtOAc = 2/1, R_{f} 0.50) gave 1.69 g (94%yield) of the iodide **13** as yellow oil. ¹H-NMR (CDCl₃): 1.15-1.34 (m, 1 H), 1.37 (s, 3H), 1.46 (s, 3H), 1.43-1.52 (m, 1 H), 1.63-1.80 (m, 2H), 1.85-1.98 (m, 2H), 3.20-3.32 (m, 2H), 3.32 (s, 3H), 3.36 (s, 3H), 3.92-4.05 (m, 2H), 4.51-4.58 (m, 1H). ¹³C-NMR (CDCl₃): 2.6, 19.8, 30.1, 36.4, 39.5, 39.6, 52.9, 53.6, 65.6, 68.5, 98.6, 101.6. MS (EI, 70 eV): 343 ([M - 15]⁺, 41%), 225 ([M - 133]⁺, 43%), 75 (100%). Found: C 39.14, H 5.99; calcd. for C₁₂H₂₃IO₄: C 40.24, H 6.47.

### Example 11: Enantioselective hydrogenation of functionalized β-keto ester 3 (according to scheme 1) comparing different chiral catalysts

| Entry | Catalyst | Solvent | S/C ratio | T [°C] | Time [h] | Conv [%] | Ee [%] |
|---|---|---|---|---|---|---|---|
| 1 | Ru((*R*)-BINAP)Cl₂ | CH₂Cl₂ | 200 | RT | 20 | 46 | n.d. |
| 2 | Ru((*R*)-BINAP)Cl₂ | EtOH | 500 | RT | 20 | 68 | n.d. |
| 3 | Ru((*R*)-BINAP)Cl₂ | MeOH | 500 | RT | 3 | 100 | 98.2 (*R*) |
| 4 | Ru((*R*)-BINAP)Cl₂ | MeOH | 1.000 | RT | 10 | 100 | 98.2 (*R*) |
| 5 | Ru((*R*)-BINAP)Cl₂ | MeOH | 2.000 | RT | 24 | 35 | n.d. |
| 6 | Ru((*R*)-BINAP)Cl₂ | MeOH | 2.000 | 50 | 1 | 100 | 97.8 (*R*) |
| 7 | Ru((*R*)-BINAP)Cl₂ (10 bar) | MeOH | 2.000 | 50 | 4 | 100 | 98.4 (*R*) |
| 8 | Ru((*R*)-BINAP)Cl₂ | MeOH | 10.000 | 70 | 1 | 100 | 97.0 (*R*) |
| 9 | Ru((*R*)-BINAP)Cl₂ | MeOH | 20.000 | 70 | 8 | 100 | 95.8 (*R*) |
| 10 | Ru((*R*)-BINAP)Cl₂ (100 bar) | MeOH | 20.000 | 100 | 0.3 | 73 | 90.0 (*R*) |
| 11 | Ru((*R*)-Tol-BINAP)Cl₂ | CH₂Cl₂ | 200 | 45 | 6 | 82 | n.d. |
| 12 | Ru((*R*)-Tol-BINAP)Cl₂ | MeOH | 500 | RT | 3 | 100 | 98.2 (*R*) |
| 13 | [Rh((*R,S*)-Josiphos)COD]BF₄ | AcOEt | 500 | RT | 2 | 100 | 15.2 (*R*) |
| 14 | [Rh((*R,S*)-Josiphos)COD]BF₄ | AcOEt | 1000 | RT | 20 | 88 | n.d. |
| 15 | [Rh(*R,R*)-DIOP)COD]BF₄ | AcOEt | 500 | RT | 4 | 100 | 7.6 (*S*) |
| 16 | [Rh((*R,R*)-DIOP)COD]BF₄ | AcOEt | 1000 | RT | 20 | 64 | n.d. |
| 17 | [Rh(*R*)-BINAP)COD]BF₄ | AcOEt | 500 | RT | 20 | 100 | 58.6 (*S*) |
| 18 | [Rh((S,S)-Chiraphos)COD]BF₄ | AcOEt | 500 | RT | 20 | 100 | 29.4 (*S*) |
| 19 | [Rh((S,S)-Deguphos)COD]BF₄ | AcOEt | 500 | RT | 18 | <5 | n.d. |
| 20 | [Rh((S,S)-Me-DuPhos)COD]BF₄ | AcOEt | 200 | 45 | 6 | 78 | n.d. |
| 21 | [Rh((R,R)-Me-catASiumM)COD]BF₄ | AcOEt | 500 | RT | 10 | 100 | 6.4 (*S*) |

Reactions have been performed at an initial H₂-pressure of 50 bar, when not indicated otherwise.

### Example 12: (17) (compound of formula IX)

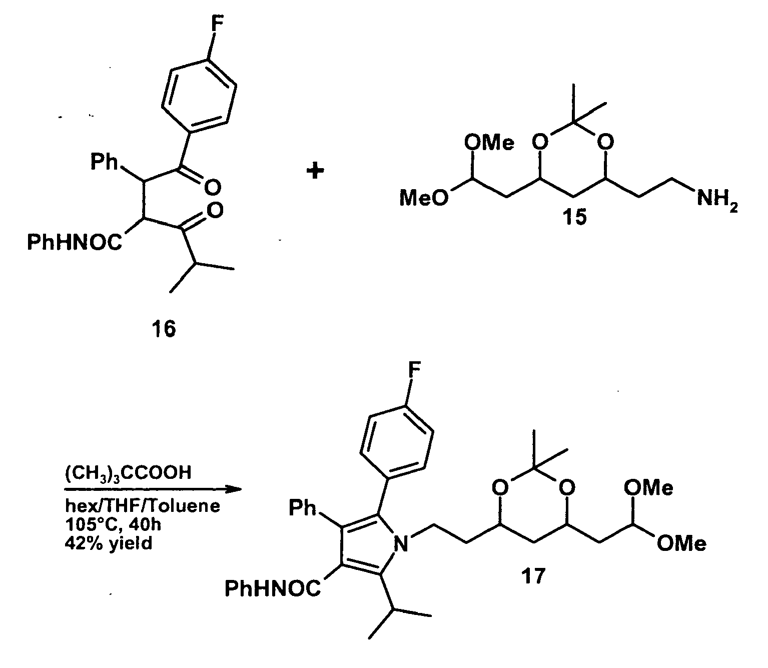

A solution of the diketone **16** (0.207 g, 0.5 mmol), amine **15** (0.124 g, 0.5 mmol) and pivalic acid (0.051 g, 0.5 mmol) in 8 ml of the heptane/THF/toluene (10/5/6) mixture was stirred under reflux (105°C oil bath) for 40 h. Evaporation of the solvent in vacuum followed by column chromatography of the residue (silica, hexane/EtOAc = 3/1, R_{f} 0.20) gave pyrrole **17** as white solid (0.130 g, 42% yield). ¹H-NMR (CDCl₃): 0.98-1.12 (m, 1H), 1.21-1.29 (m, 1H), 1.31 (s, 3H), 1.35 (s, 3H), 1.53 (d, *J* = 7.3 Hz, 6H), 1.60-1.78 (m, 4H), 3.31 (s, 3H), 3.33 (s, 3H), 3.57 (septet, *J =* 7.3 Hz, 1 H), 3.60-3.72 (m, 1 H), 3.75-3.93 (m, 2H), 4.01-4.15 (m, 1H), 4.47-4.53 (m, 1H), 6.86 (br., 1H), 6.94-7.10 (m, 5H), 7.11-7.24 (m, 9H). ¹³C-NMR (CDCl₃): 20.2, 22.0, 22.2, 26.5, 30.5, 36.9, 38.6, 39.9, 41.3, 53.3, 54.1, 65.8, 66.9, 98.9, 102.0, 115.7, 115.8 (d, *J =* 18 Hz), 120.0, 123.9, 127.0, 128.6, 129.1, 129.2, 130.9, 133.6 (d, *J* = 9 Hz), 135.1, 136.1, 138.8, 141.9, 162.6 (d, *J* = 248 Hz), 165.3.

### Example 13: (12) (Compound of formula VIII)

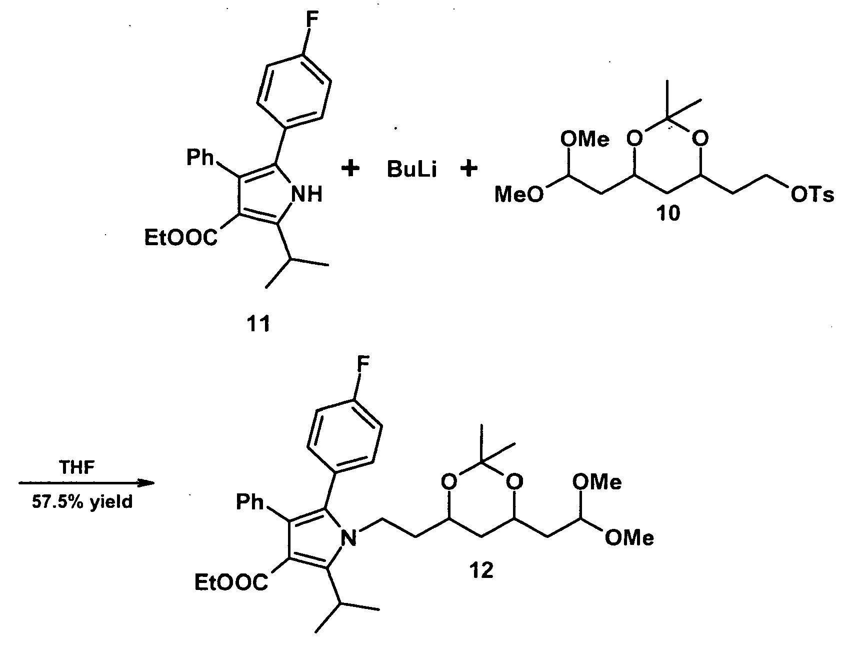

0.200 ml of 1.6M BuLi (0.32 mmol) was added dropwise to a stirred solution of the pyrrole 11 (0.105 g, 0.3 mmol) in 8 ml of abs. THF at -78°C. The solution was stirred for 1 h at the same temperature, warmed to RT and 0.141 g (0.35 mmol) of the tosylate **10** was added neat. The mixture was refluxed under argon for 24 h, concentrated in vacuum, and the yellow semi-solid residue was purified by column chromatography (silica, hexane/EtOAc = 2/1, R_{f} 0.40) to obtain **12** as colorless paraffin (0.100 g, 57.5% yield). ¹H-NMR (CDCl₃): 0.93 (t, *J* = 7.1 Hz, 3H), 0.98-1.09 (m, 1H), 1.19-1.27 (m, 1 H), 130 (s, 3H), 1.33 (s, 3H) 1.47 (d, *J* = 7.1 Hz, 6H), 1.57-1.76 (m, 4H), 3.30 (s, 3H), 3.33 (s, 3H), 3.48 (septet, *J* = 7.1 Hz, 1H), 3.60-3.68 (m, 1H), 3.73-3.91 (2H), 3.98-4.07 (m, 1 H), 4.02 (q, *J* = 7.1 Hz, 2H), 4.47-4.51 (m, 1H), 6.93-6.99 (m, 2H), 7.01-7.05 (m, 2H), 7.07-7.16 (m, 5H). ¹³C-NMR (CDCl₃): 14.1, 20.2, 21.7, 21.8, 26.4, 30.4, 36.9, 38.5, 39.9, 41.3, 53.3, 54.0, 60.1, 65.7, 66.9, 98.9, 102.0, 111.8, 115.6 (d, *J* = 21 Hz), 121.6, 125.9, 127.6, 128.8 (d, *J* = 3 Hz), 129.5, 130.7, 133.7 (d, *J* = 9 Hz), 136.3, 142.8, 162.5 (d, *J* = 248 Hz), 167.1.

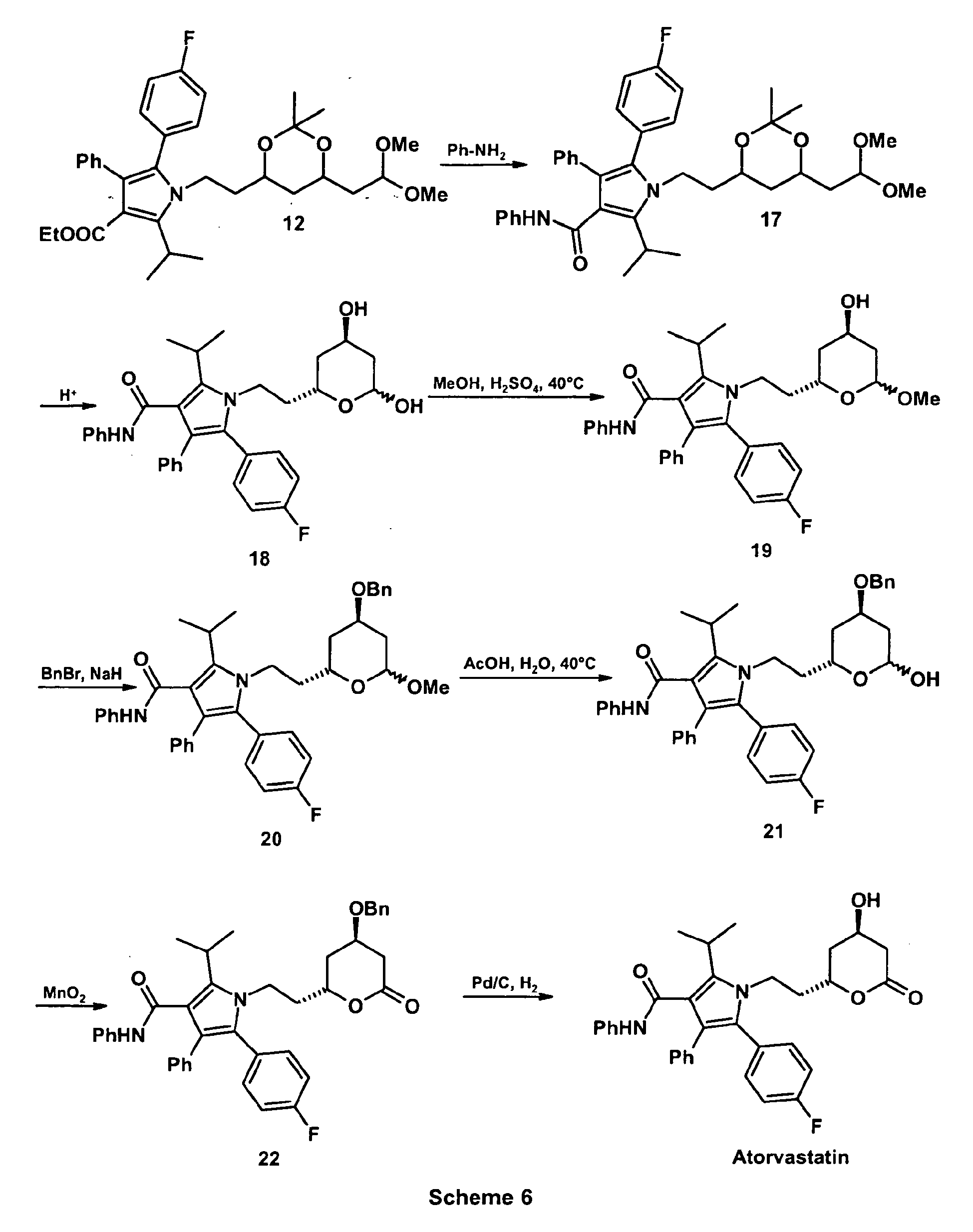

The each transformation was monitored by HPLC or GC analysis and the structure of all products was determined using LC-MS system.

### Example 14

**(2*R*,4*R*)-1-{2-[4-(tert-Butyldimethylsilyloxy)-6-oxotetrahydro-2-pyranyl]-ethyl}-5-(4-fluorophenyl)-2-isopropyl-4-phenyl-1*H*-pyrrole-3-carboxylic acid phenylamide (23)** (synthesis from Atorvastatin) was prepared in 92% yield according to WO 2005/012246 A1. 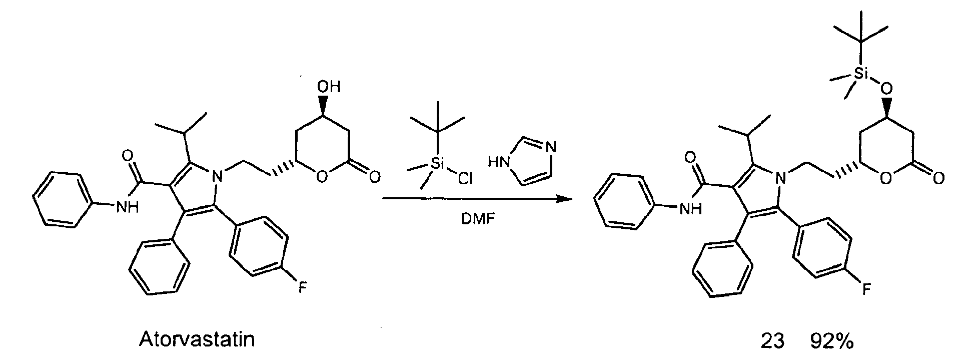 LC-MS (ESI+) m/z 655 (M+1).

### Example 15:

**(2*R*,4*R*)-1-{2-[4-(tert-Butyldimethylsilyloxy)-6-hydroxytetrahydro-2-pyranyl]-ethyl}-5-(4-fluorophenyl)-2-isopropyl-4-phenyl-1*H*-pyrrole-3-carboxylic acid phenylamide (24)** was prepared 99% yield according to WO 2005/012246 A1 with the difference that 30% more of DIBAL was used. 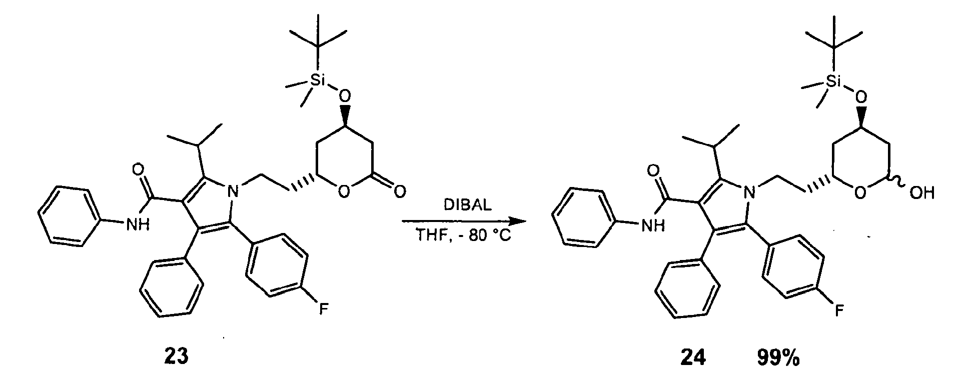 LC-MS (ESI+) m/z 657 (M+1), 639, 507, 414.

### Example 16:

**(2*R*,4*R*)-1-[2-(4,6-Dihydroxytetrahydro-2-pyranyl)-ethyl]-5-(4-fluorophenyl)-2-isopropyl-4-phenyl-1*H*-pyrrole-3-carboxylic acid phenylamide (18)** (synthesis from **24**).

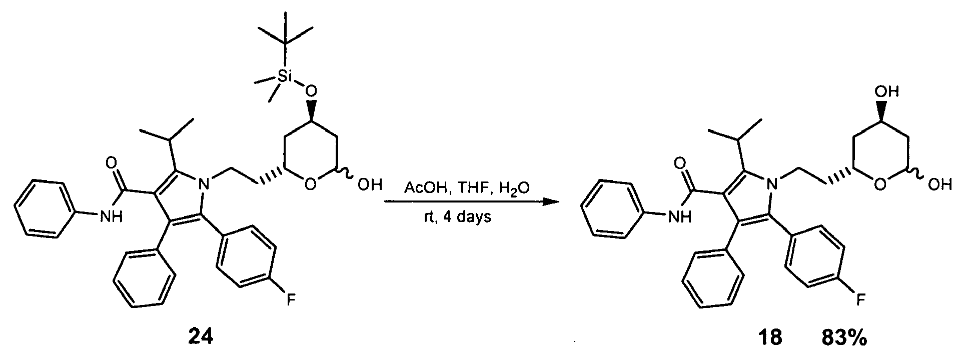

The TBDMS lactol **24** (1.0 g, 1.5 mmol) was dissolved in THF (10 ml) and acetic acid (30 ml) and water (10 ml) were added. The mixture was stirred at room temperature for 4 days. Cooled reaction mixture was neutralized with 1 M NaOH to pH 7 - 8 at room temperature and extracted with methylene chloride (3 x 50 ml). Organic layer was washed with saturated NaHCO₃ solution (2 x 20 ml) and brine (50 ml), dried over anhydrous sodium sulfate and solvent was evaporated at reduced pressure to yield 0.68 g (83 %) of crude lactol **18.** LC-MS (ESI+) m/z 543 (M+1), 507, 414, 388.

### Example 17:

**(2*R*,4*R*)-1-[2-(4,6-Dihydroxytetrahydro-2-pyranyl)-ethyl]-5-(4-fluorophenyl)-2-isopropyl-4-phenyl-1*H*-pyrrole-3-carboxylic acid phenylamide (18)** (synthesis from Atorvastatin).

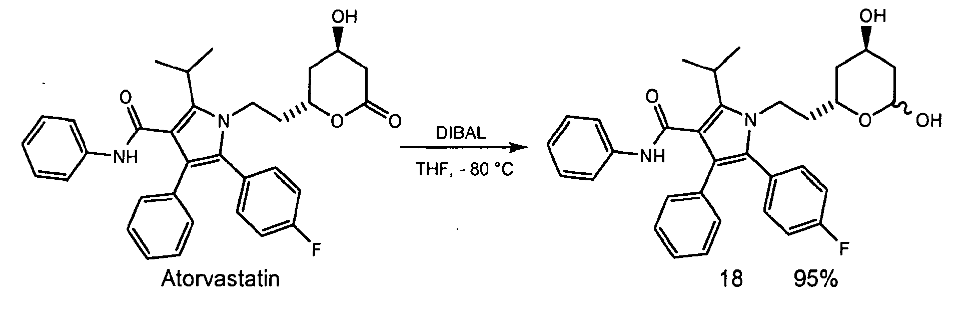

The atorvastatin lactone (21.6 g, 0.04 mol) was dissolved in anhydrous methylene chloride (500 ml) and then cooled to - 78 °C with help of dry ice. DIBAL (80 ml, 1M solution in toluene) was added dropwise at the same temperature and stirring continued for 1 hour. The mixture was then quenched with 10 % solution of Rochelle's salt (100 ml) and allowed to warm to room temperature. After addition of further methylene chloride (300 ml) and water (200 ml) concentrated HCl (50 ml) was added. Organic phase was separated and water phase was extracted with methylene chloride (2 x 200 ml). The combined organic phases were washed with 1 M HCl solution (2 × 100 ml), saturated NaHCO₃ solution (2 × 100 ml) and brine (100 ml) and dried over anhydrous Na₂SO₄. Solvent was removed under reduced pressure to yield 20.6 g (95 %) of pure lactol **18**. LC-MS (ESI+) m/z 543 (M+1), 507,414,388.

### Example 18:

**(2*R*,4*R*)-5-(4-Fluorophenyl)-1-[2-(4-hydroxy-6-methoxytetrahydro-2-pyranyl)-ethyl]-2-isopropyl-4-phenyl-1*H*-pyrrole-3-carboxylic acid phenylamide (19)**

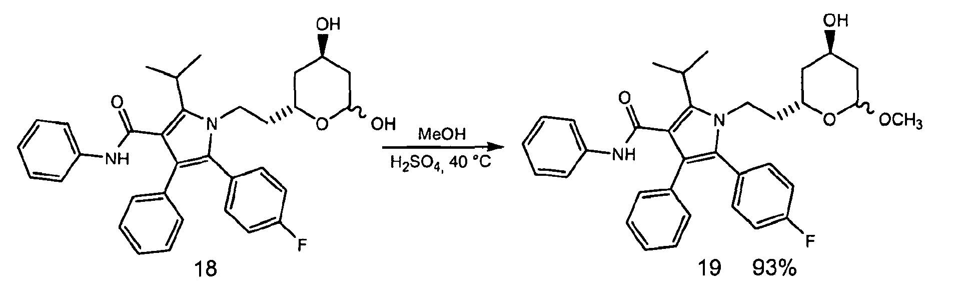

The atorvastatin lactol **18** (8.0 g, 14.7 mmol) was dissolved in methanol (100 ml) and concentrated sulfuric acid (0.1 ml) was added dropwise. Stirred reaction mixture was heated at 40 °C for 2 h and then volume of solvent was reduced to about 30 ml at reduced pressure. Water (100 ml) was added at room temperature and product was extracted with methylene chloride (3 x 100 ml). Organic phases were washed with saturated NaHCO₃ solution (2 × 100 ml) and brine (100 ml) and dried over anhydrous Na₂SO₄. Solvent was removed under reduced pressure to yield 7.6 g (93 %) of lactol methyl acetal **19** as a white solid. LC-MS (ESI+) m/z 557 (M+1), 525, 507, 322.

### Example 19:

### (2R,4R)-1-[2-(4-Benzyloxy-6-methoxytetrahydro-2-pyranyl)-ethyl]-5-(4-fluoro-phenyl)-2-isopropyl-4-phenyl-1H-pyrrole-3-carboxylic acid phenylamide (20)

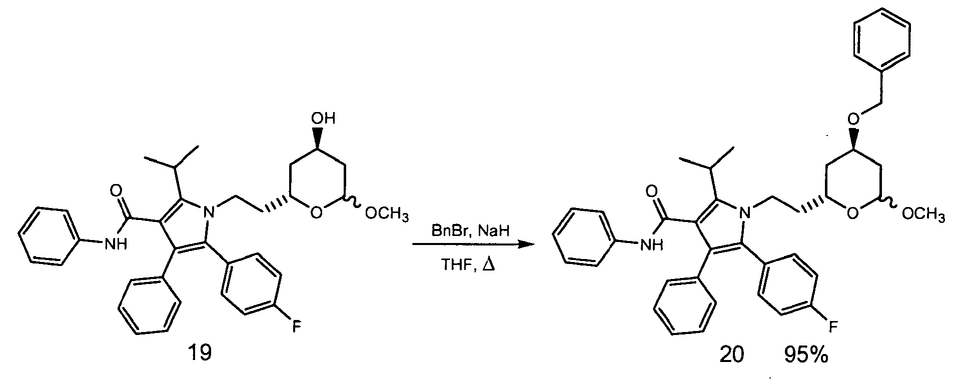

The atorvastatin lactol methyl acetal **19** (7.5 g, 13.5 mmol) dissolved in anhydrous THF (50 ml) was added dropwise to stirred suspension of sodium hydride (0.54 g, 60 % in mineral oil, 13.5 mmol, washed previously with anhydrous n-hexane) in anhydrous THF (200 ml). After half an hour of stirring at room temperature benzylbromide (1.6 ml, 13.5 mmol) was added dropwise and mixture was stirred for 2 hours at the same temperature followed by reflux for 10 hours. To cooled mixture was added methanol (10 ml), water (200 ml) and diethylether (200 ml). Phases were separated. Water phase was extracted with diethyl ether (3 x 100 ml). Combined organic phases were washed with 1 M HCl solution (2 × 100 ml), saturated NaHCO₃ solution (2 × 100 ml) and brine (100 ml) and dried over anhydrous Na₂SO₄. Solvent was removed under reduced pressure to yield 8.3 g (95 %) of benzyl lactol methyl acetal **20** as a brownish solid. LC-MS (ESI+) m/z 647 (M+1), 597.

### Example 20:

### (2R,4R)-1-[2-(4-Benzyloxy-6-hydroxytetrahydro-2-pyranyl)-ethyl]-5-(4-fluorophenyl)-2-isopropyl-4-phenyl-1H-pyrrole-3-carboxylic acid phenylamide (21)

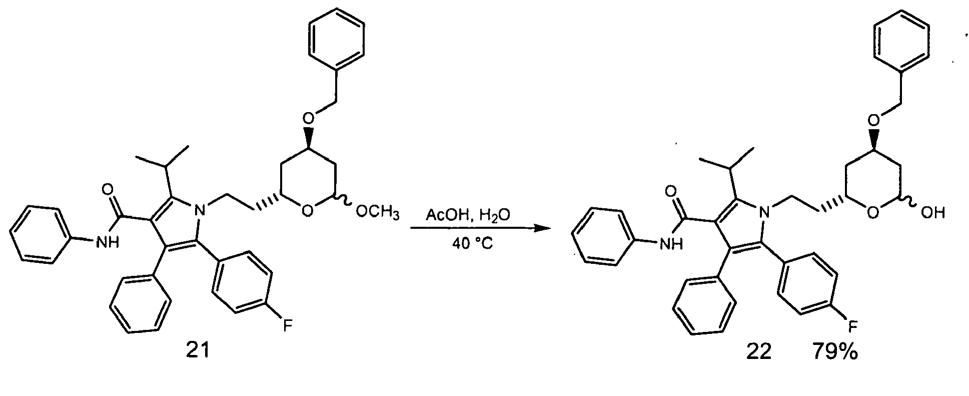

The diprotected lactol **20** (4 g, 6.2 mmol) was dissolved in acetic acid (100 ml) and water (10 ml) was added. The reaction mixture was heated to 40 °C for 24 hours. Cooled reaction mixture was neutralized with 1 M NaOH to pH 7 - 8 at room temperature and extracted with methylene chloride (3 x 100 ml). Organic layer was washed with saturated NaHCO₃ solution (2 x 100 ml) and brine (100 ml), dried over anhydrous sodium sulfate and solvent was evaporated at reduced pressure to yield 3.5 g (90 %) of product **21**. LC-MS (ESI+) m/z 633 (M+1), 597.

### Example 21:

### (2R,4R)-5-(4-Fluorophenyl)-1-[2-(4-benzyloxy-6-oxotetrahydropyran-2-yl)-ethyl]-2-isopropyl-4-phenyl-1H-pyrrole-3-carboxylic acid phenylamide (22)

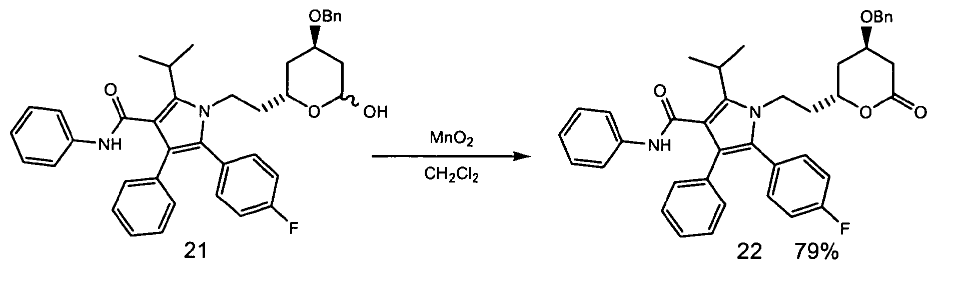

(2*R*,4*R*)-1-[2-(4-benzyloxy-6-hydroxytetrahydro-2-pyranyl)-ethyl]-5-(4-fluorophenyl)-2-iso-propyl-4-phenyl-1*H*-pyrrole-3-carboxylic acid phenylamide **21** (atorvastatin unprotected lactol, 0.315 g, 0.5 mmol) was dissolved in dichloromethane (60 ml) and activated MnO₂ (2.8 g, 32.2 mmol, 64 eq.) was added and stirred at room temperature for 2 hours. The reaction mixture was filtrated and solid part was washed sufficiently with dichloromethane (100 ml). The solvent from combined filtrates was removed under reduced pressure to yield 0.25 g (79 %) of the product **22** as a white - yellowish solid. LC-MS (ESI+) m/z 631 (M+1).

## Claims

1. Process for the preparation of a compound of the formula I 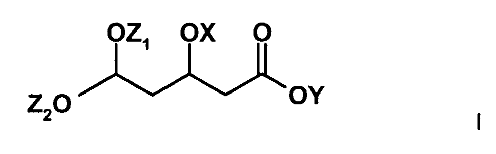 or a salt thereof, wherein Z₁ is a hydroxy protecting group, Z₂ is a hydroxy protecting group, X is H or a hydroxy protecting group, and Y is H or a carboxy protecting group, which process comprises the steps of
a) reacting a compound of the formula II 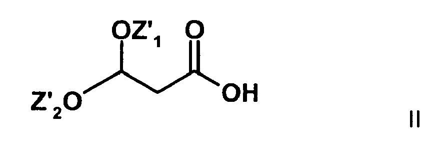 wherein Z'₁ is a hydroxy protecting group and Z'₂ is a hydroxy protecting group, with N,N'-carbonyldiimidazole (Im₂CO) and a compound of the formula III 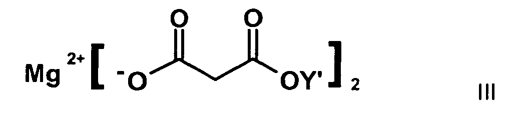 wherein Y' is a carboxy protecting group, to a compound of the formula IV 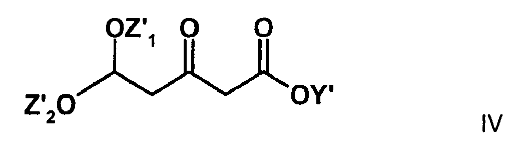 wherein Z'₁, Z'₂ and Y' are defined as above,
b) reducing the compound of the formula IV to obtain a compound of the formula V 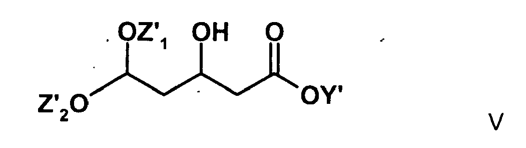 wherein Z'₁, Z'₂ and Y' are defined as above, and
c) optionally deprotecting the hydroxy groups and/or the carboxy group of the compound of the formula V, optionally protecting the free hydroxy group of the compound of the formula V and/or optionally converting the resulting compound into a salt thereof.

2. Process according to claim 1, wherein Z₁, Z₂, Z'₁ and Z'₂ are methyl, X is H and Y' can be independent from each other an alkyl, aryl or aralkyl, preferably methyl, ethyl, iso-propyl or tert-butyl.

3. Process according to any of the preceding claims, wherein the reduction step (b) is a hydrogenation which is carried out in the presence of a chiral catalyst.

4. Process according to claim 3, wherein the chiral catalyst is selected from chiral iridium, rhodium and ruthenium complexes.

5. Process according to claim 3 or 4, wherein the chiral catalyst is Ru((R)-BINAP)Cl₂.

6. Process for preparing a compound of the formula I 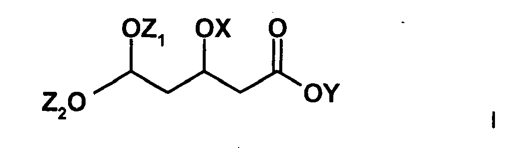 wherein Z₁ is a hydroxy protecting group, Z₂ is a hydroxy protecting group, X is H or a hydroxy protecting group and Y is H or a carboxy protecting group, which process comprises the step of homogenous achiral or chiral enantioselective hydrogenation of a compound of the formula IV 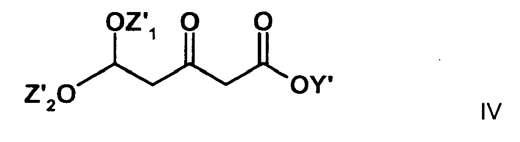 wherein Z'₁ is a hydroxy protecting group, Z'₂ is a hydroxy protecting group and Y' is a carboxy protecting group, and optionally deprotecting and/or protecting any protected or unprotected group.

7. Process according to any of claims 1-5, which comprises the steps of claim 6.

8. Process according to any of claims 1-5 or 7, further comprising the step of reacting the compound of the formula I with a compound of formula XV 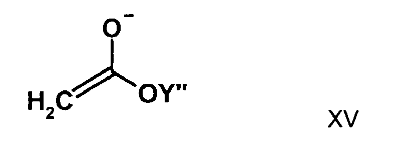 wherein Y" is a carboxy protecting group to obtain a compound of formula VI 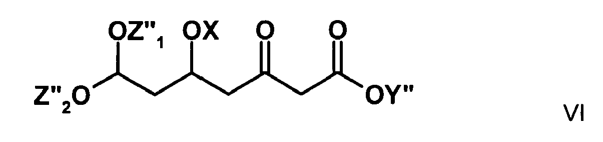 or a salt thereof, wherein Z"₁ is a hydroxy protecting group, Z"₂ is a hydroxy protecting group, X is H or a hydroxy protecting group and Y" is defined as above and optionally deprotecting and/or protecting any protected or unprotected group.

9. Process according to claim 8, further comprising the step of achiral or chiral enantioselective hydrogenation of a compound of formula VI and optionally deprotecting and/or protecting any protected or unprotected group to obtain a compound of formula VIa 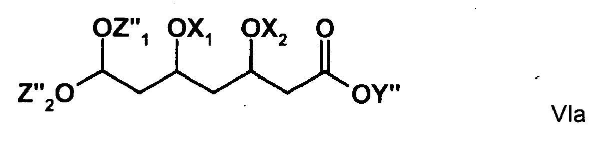 or a salt thereof, wherein Z"₁ is a hydroxy protecting group, Z"₂ is a hydroxy protecting group, X₁ and X₂ can be independent from each other a hydroxy protecting group or X₁ and X₂ taken together may form a ring with the oxygen and intermediate carbon atoms to which they are attached and Y" is defined as in claim 8.

10. Process according to claim 9, further comprising the step of hydrogenation and/or group modification of a compound of formula VIa to obtain a compound of formula VIb 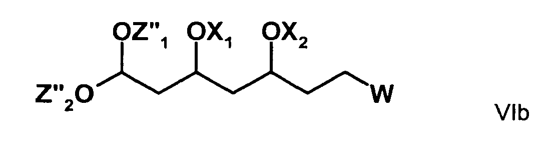 wherein Z"₁ is a hydroxyl protecting group, Z"₂ is a hydroxyl protecting group, X₁ and X₂ can be independent from each other a hydroxyl protecting group or X₁ and X₂ taken together may form a ring with the oxygen and intermediate carbon atoms to which they are attached and W is NH₂, N₃, OH, OW₁ or a leaving group and W₁ is a hydroxyl protecting group.

11. Process according to claim 10, further comprising the step of reacting the compound of the formula VIb, wherein W is a leaving group, with a compound of the formula VII 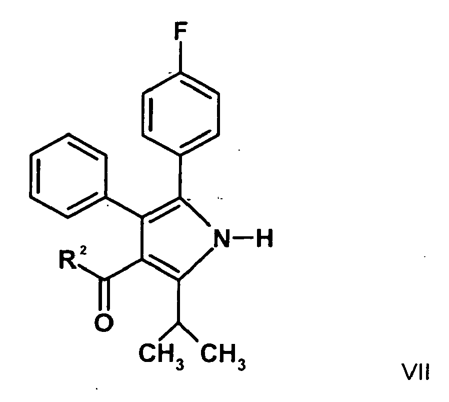 wherein R² is -OR³, -NR⁴R⁵, -NR¹⁰CONR¹¹R¹², -NR¹³OR¹⁴, -ONR¹⁵R¹⁶ or halogen, R³, R⁴, R⁵, R⁶, R⁷, R⁸, R⁹, R¹⁰, R¹¹, R¹², R¹³, R¹⁴, R¹⁵ and R¹⁶ are independently selected from hydrogen or a straight, branched and/or cyclic, saturated or unsaturated C₁₋₁₀ alkyl residue or aryl residue, both residues being optionally substituted with 1-3 optionally protected hydroxy or carboxy groups, 1-3 -OR⁷ residues, 1-3 -NR⁸R⁹ residues and/or 1-3 halogen atoms, the C₁₋₁₀ alkyl residue optionally comprising 1-3 oxygen atoms, 1-3 nitrogen atoms and/or 1-3 -NR⁶- residues, the C₁₋₁₀ alkyl residue further optionally comprising or being substituted with 1 or 2 aryl residues, to obtain a compound of the formula VIII 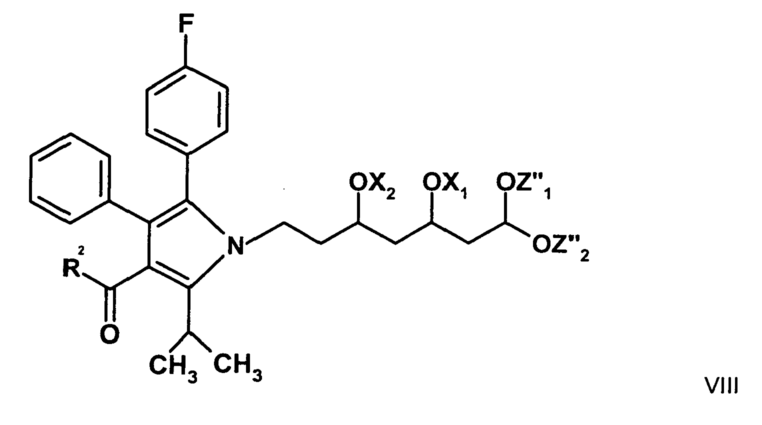 wherein R² is defined as above and X₁, X₂, Z"₁ and Z"₂ are defined as in claim 10.

12. Process according to claim 10, further comprising the step of reacting the compound of the formula VIb, wherein W is NH₂, with a compound of the formula XVII 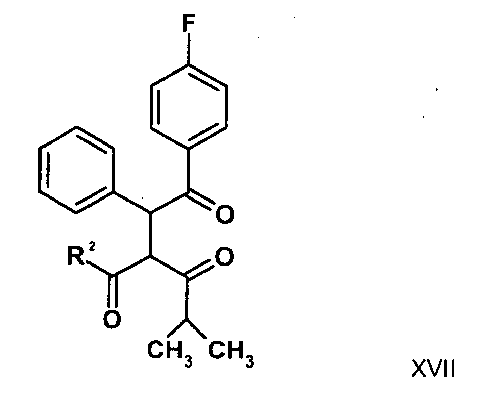 wherein R₂ is defined as in claim 11 to obtain a compound of the formula VIII 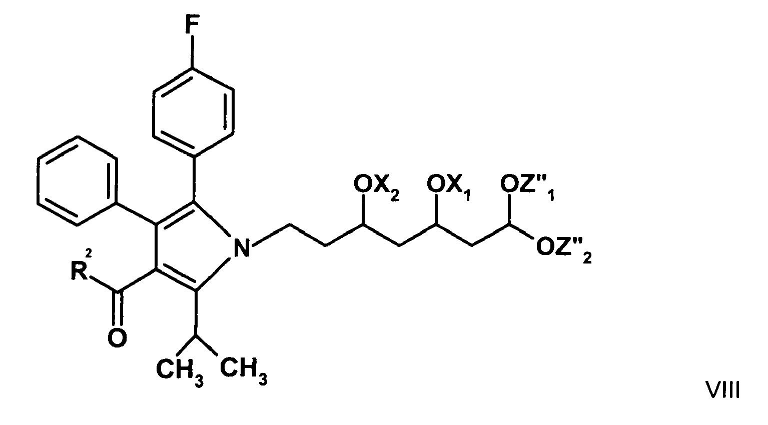 wherein R² is defined as above and X₁, X₂, Z"₁ and Z"₂ are defined as in claim 10.

13. Process according to claim 11 or 12, further comprising the step of converting and optionally deprotecting and/or protecting any protected or unprotected group of a compound of the formula VIII to obtain a compound of the formula IX 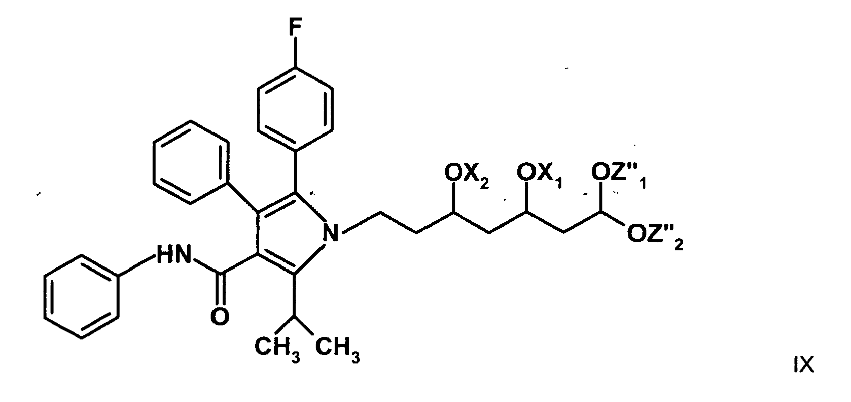 wherein X₁, X₂, Z"₁ and Z"₂ are defined as in claim 10.

14. Process of claim 13, further comprising the step of hydrating and optionally deprotecting and/or protecting any protected or unprotected group of a compound of the formula IX to obtain a compound of the formula X 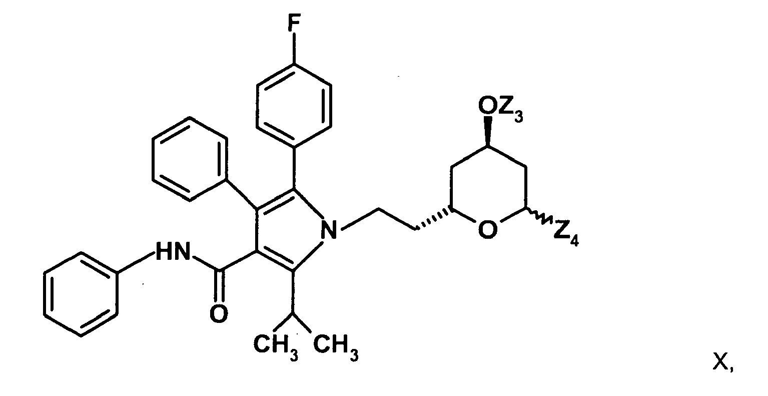 wherein Z₃ is H or a hydroxy protecting group, Z₄ is =O, OH or OZ₅ and Z₅ is a hydroxy protecting group.

15. Process according to any of claims 1-14, wherein the compound of the formula I has the formula I' 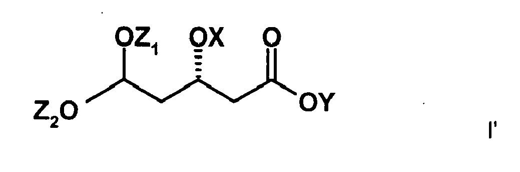 wherein Z₁, Z₂, X and Y are defined as in claim 1.

16. Process for preparing a compound of the formula VI 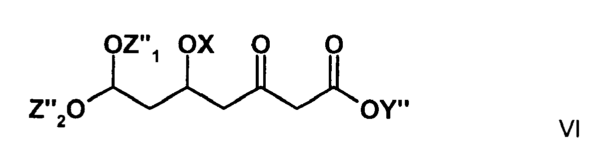 or a salt thereof, wherein Z"₁ is a hydroxy protecting group, Z"₂ is a hydroxy protecting group, X is H or a hydroxy protecting group and Y" is a carboxy protecting group, which process comprises the step of reacting the compound of formula I 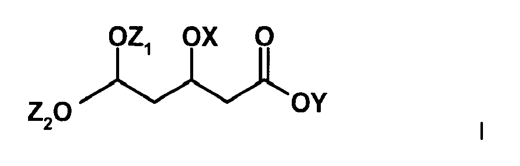 or a salt thereof, wherein Z₁ is a hydroxy protecting group, Z₂ is a hydroxy protecting group, X is H or a hydroxy protecting group, and Y is H or a carboxy protecting group, with a compound of formula XV 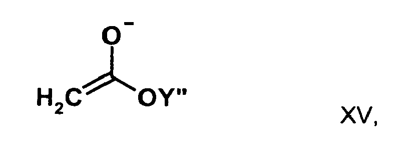 wherein Y" is a carboxy protecting group, and optionally deprotecting and/or protecting any protected or unprotected group.

17. Process for preparing a compound of the formula VIa 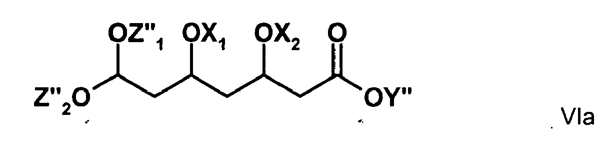 or a salt thereof, wherein Z"₁ is a hydroxy protecting group, Z"₂ is a hydroxy protecting group, X₁ and X₂ can be independent from each other a hydroxy protecting group or X₁ and X₂ taken together may form a ring with the oxygen and intermediate carbon atoms to which they are attached and Y" is a carboxy protecting group, which process comprises the step of achiral or chiral enantioselective hydrogenation of a compound of formula VI 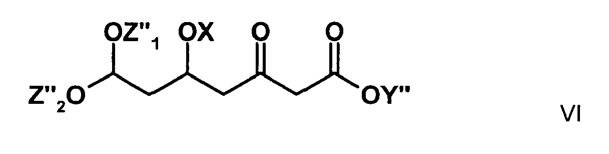 or a salt thereof, wherein Z"₁ is a hydroxy protecting group, Z"₂ is a hydroxy protecting group, X is H or a hydroxy protecting group and Y" is defined as above and optionally deprotecting and/or protecting any protected or unprotected group.

18. Process for preparing a compound of the formula VIb 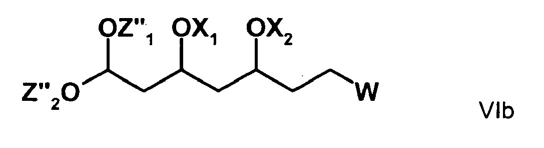 wherein Z"₁ is a hydroxyl protecting group, Z"₂ is a hydroxyl protecting group, X₁ and X₂ can be independent from each other a hydroxyl protecting group or X₁ and X₂ taken together may form a ring with the oxygen and intermediate carbon atoms to which they are attached and W is NH₂, N₃, OH, OW₁ or a leaving group and W₁ is a hydroxyl protecting group, which process comprises the step of hydrogenation and/or group modification of a compound of formula VIa 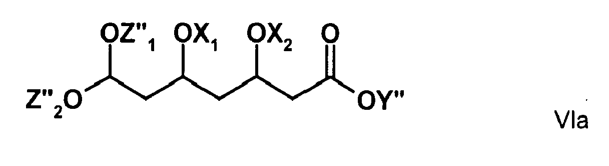 or a salt thereof, wherein Z"₁ is a hydroxy protecting group, Z"₂ is a hydroxy protecting group, X₁ and X₂ can be independent from each other a hydroxy protecting group or X₁ and X₂ taken together may form a ring with the oxygen and intermediate carbon atoms to which they are attached and Y" is a carboxy protecting group.

19. Process for preparing a compound of the formula VIII 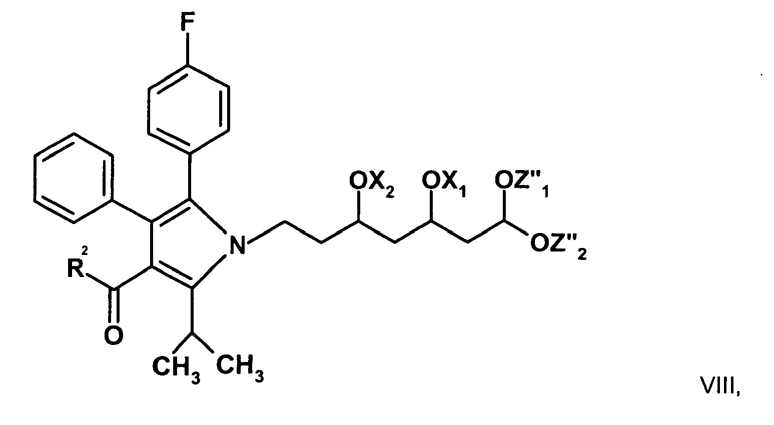 wherein R² is -OR³, -NR⁴R⁵, -NR¹⁰CONR¹¹R¹², -NR¹³OR¹⁴, -ONR¹⁵R¹⁶ or halogen, R³, R⁴, R⁵, R⁶, R⁷, R⁸, R⁹, R¹⁰, R¹¹, R¹², R¹³, R¹⁴, R¹⁵ and R¹⁶ are independently selected from hydrogen or a straight, branched and/or cyclic, saturated or unsaturated C₁₋₁₀ alkyl residue or aryl residue, both residues being optionally substituted with 1-3 optionally protected hydroxy or carboxy groups, 1-3 -OR⁷ residues, 1-3 -NR⁸R⁹ residues and/or 1-3 halogen atoms, the C₁₋₁₀ alkyl residue optionally comprising 1-3 oxygen atoms, 1-3 nitrogen atoms and/or 1-3 -NR⁶ residues, the C₁₋₁₀ alkyl residue further optionally comprising or being substituted with 1 or 2 aryl residues, and X₁, X₂, Z"₁ and Z"₂ are defined as in claim 10, which process comprises reacting a compound of the formula VII 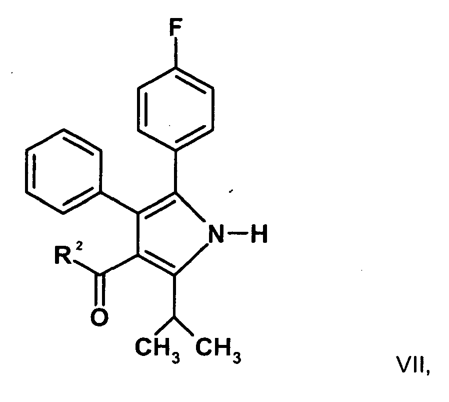 wherein R² is defined as above, with a compound of the formula VIb 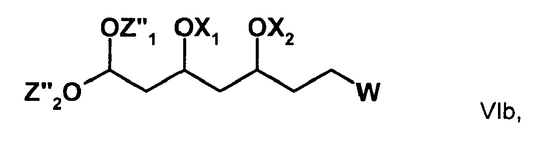 wherein W is a leaving group and X₁, X₂, Z"₁ and Z"₂ are defined as above.

20. Process for preparing a compound of the formula VIII 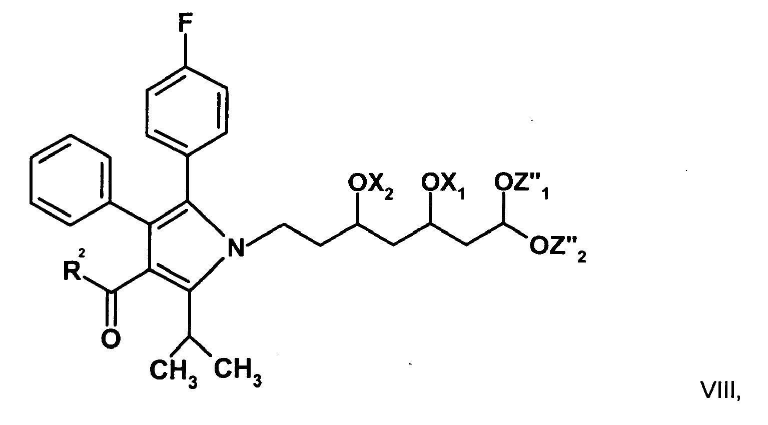 wherein R² is -OR³, -NR⁴R⁵, -NR¹⁰CONR¹¹R¹², -NR¹³OR¹⁴, -ONR¹⁵R¹⁶ or halogen, R³, R⁴, R⁵, R⁶, R⁷, R⁸, R⁹, R¹⁰, R¹¹, R¹², R¹³, R¹⁴, R¹⁵ and R¹⁶ are independently selected from hydrogen or a straight, branched and/or cyclic, saturated or unsaturated C₁₋₁₀ alkyl residue or aryl residue, both residues being optionally substituted with 1-3 optionally protected hydroxy or carboxy groups, 1-3 -OR⁷ residues, 1-3 -NR⁸R⁹ residues and/or 1-3 halogen atoms, the C₁₋₁₀ alkyl residue optionally comprising 1-3 oxygen atoms, 1-3 nitrogen atoms and/or 1-3 -NR⁶ residues, the C₁₋₁₀ alkyl residue further optionally comprising or being substituted with 1 or 2 aryl residues, and X₁, X₂, Z"₁ and Z"₂ are defined as in claim 10, which process comprises reacting a compound of the formula XVII 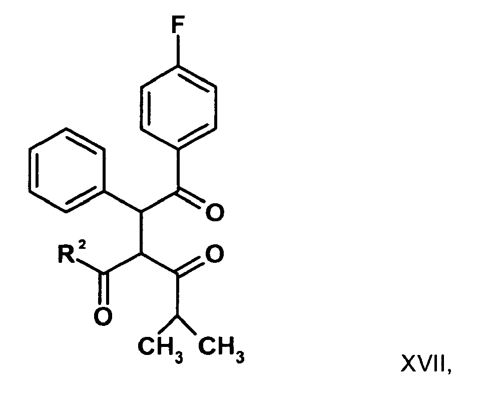 wherein R² is defined as above, with a compound of the formula VIb 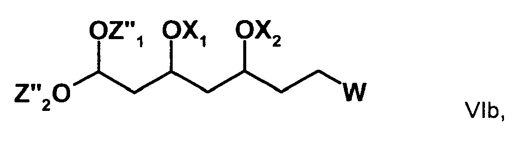 wherein W is NH₂ and X₁, X₂, Z"₁ and Z"₂ are defined as above.

21. Process for preparing a compound of the formula IX 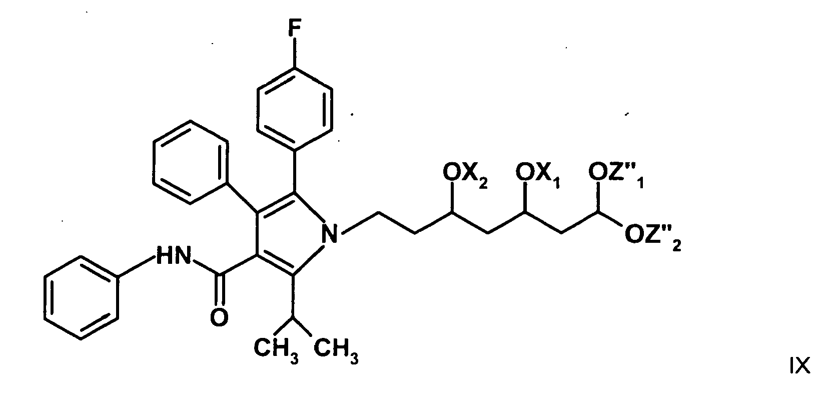 wherein X₁, X₂, Z"₁ and Z"₂ are defined as in claim 10, which process comprises the step of conversion of a compound of the formula VIII 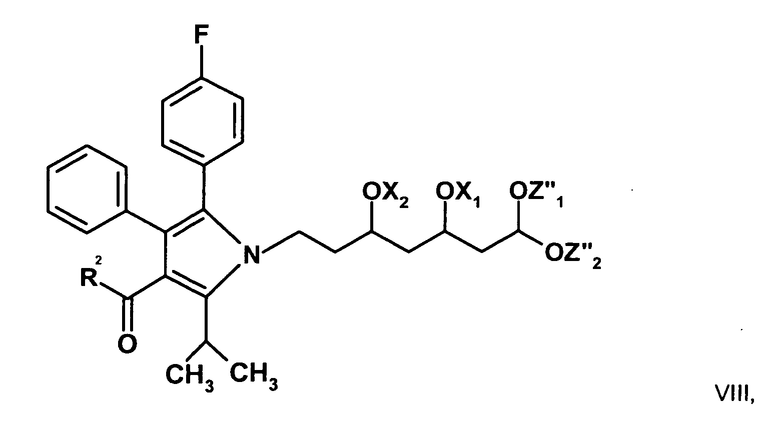 wherein R² is -OR³, -NR⁴R⁵, -NR¹⁰CONR¹¹R¹², -NR¹³OR¹⁴, -ONR¹⁵R¹⁶ or halogen, R³, R⁴, R⁵, R⁶, R⁷, R⁸, R⁹, R¹⁰, R¹¹, R¹², R¹³, R¹⁴, R¹⁵ and R¹⁶ are independently selected from hydrogen or a straight, branched and/or cyclic, saturated or unsaturated C₁₋₁₀ alkyl residue or aryl residue, both residues being optionally substituted with 1-3 optionally protected hydroxy or carboxy groups, 1-3 -OR⁷ residues, 1-3 -NR⁸R⁹ residues and/or 1-3 halogen atoms, the C₁₋₁₀ alkyl residue optionally comprising 1-3 oxygen atoms, 1-3 nitrogen atoms and/or 1-3 -NR⁶ residues, the C₁₋₁₀ alkyl residue further optionally comprising or being substituted with 1 or 2 aryl residues, and X₁, X₂, Z"₁ and Z"₂ are defined as above.

22. Process for preparing a compound of the formula X 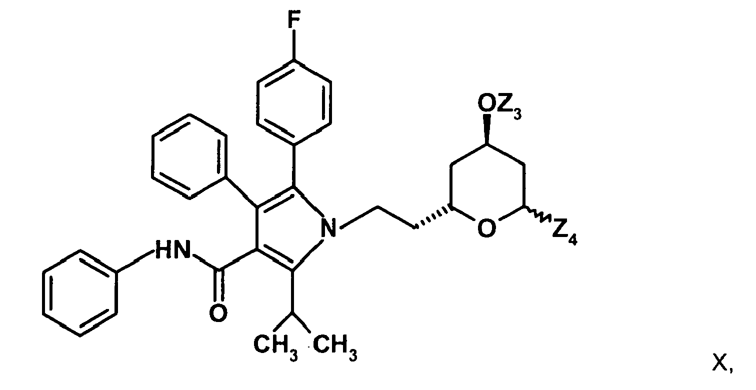 wherein Z₃ is H or a hydroxy protecting group, Z₄ is =O, OH or OZ₅ and Z₅ is a hydroxy protecting group, which process comprises the step of hydration of the compound of formula IX 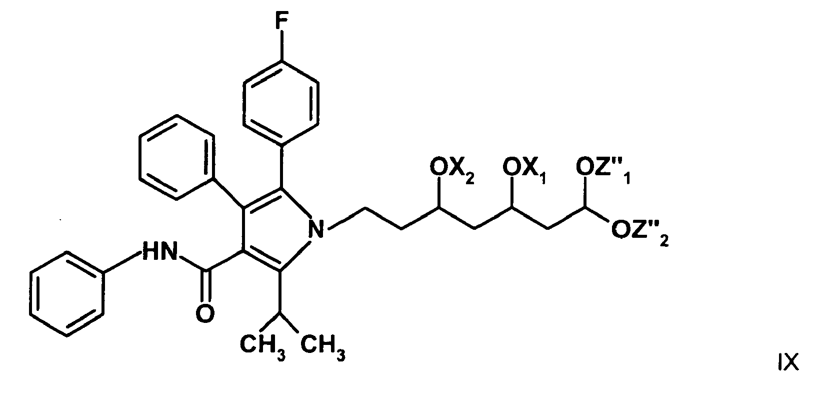 wherein X₁, X₂, Z"₁ and Z"₂ are defined as in claim 10, and optionally deprotecting and/or protecting any protected or unprotected group.

23. Compound of the formula XI 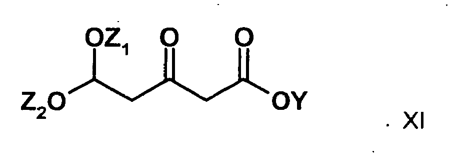 wherein Z₁ is a hydroxy protecting group, Z₂ is a hydroxy protecting group and Y is a carboxy protecting group.

24. Compound of the formula XIa

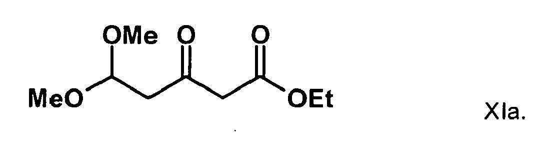

25. Compounds of the formula XIIa and XIIb

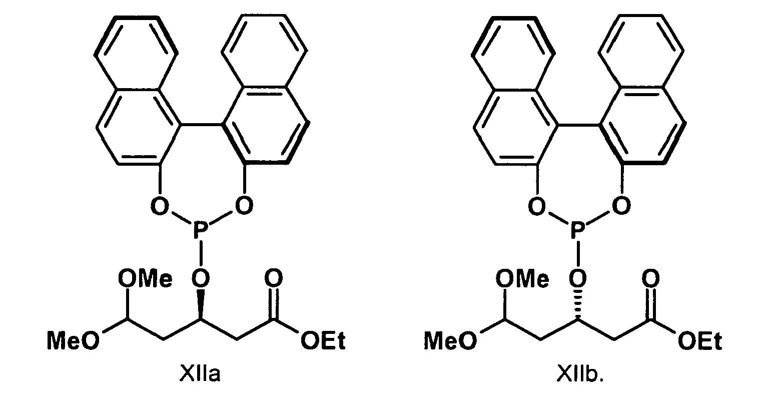

26. Compound of the formula VIII 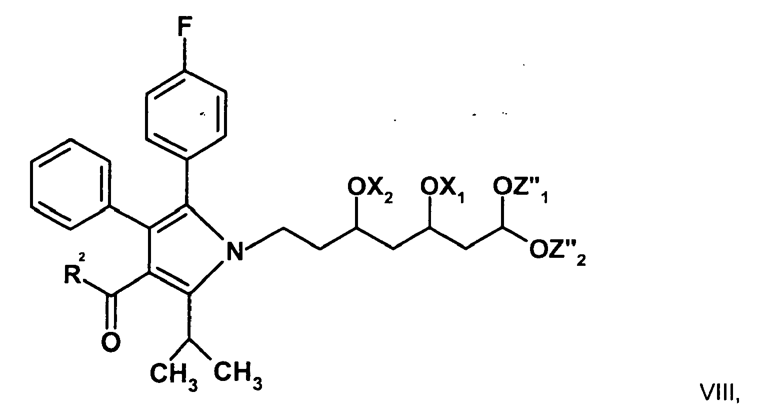 wherein R² is -OR³, -NR⁴R⁵, -NR¹⁰CONR¹¹R¹², -NR¹³OR¹⁴, -ONR¹⁵R¹⁶ or halogen, R³, R⁴, R⁵, R⁶, R⁷, R⁸, R⁹, R¹⁰, R¹¹, R¹², R¹³, R¹⁴, R¹⁵ and R¹⁶ are independently selected from hydrogen or a straight, branched and/or cyclic, saturated or unsaturated C₁₋₁₀ alkyl residue or aryl residue, both residues being optionally substituted with 1-3 optionally protected hydroxy or carboxy groups, 1-3 -OR⁷ residues, 1-3 -NR⁸R⁹ residues and/or 1-3 halogen atoms, the C₁₋₁₀ alkyl residue optionally comprising 1-3 oxygen atoms, 1-3 nitrogen atoms and/or 1-3 -NR⁶ residues, the C₁₋₁₀ alkyl residue further optionally comprising or being substituted with 1 or 2 aryl residues, and X₁, X₂, Z"₁ and Z"₂ are defined as in claim 10.

27. Compound of the formula XIII

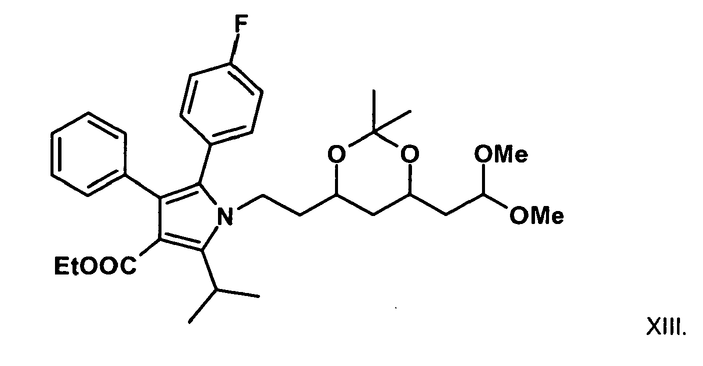

28. Compound of the formula XIV

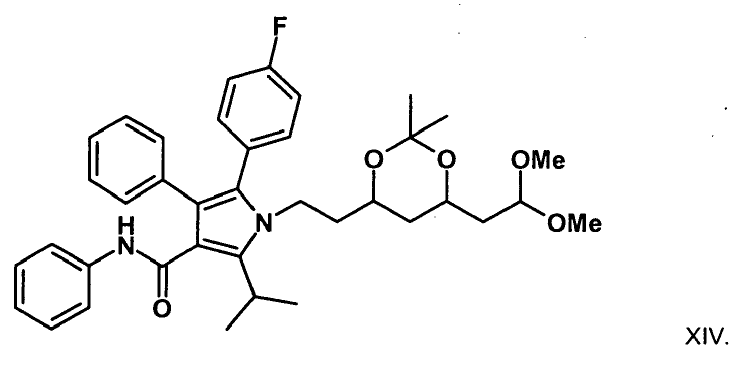

29. Compound of the formula IV 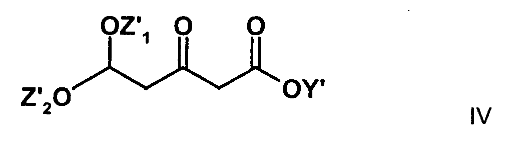 wherein Z'₁ is a hydroxy protecting group, Z'₂ is a hydroxy protecting group and Y' is a carboxy protecting group.

30. Compound of the formula V 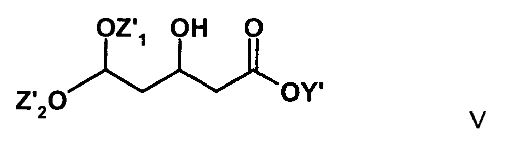 wherein Z'₁ is a hydroxy protecting group, Z'₂ is a hydroxy protecting group and Y' is a carboxy protecting group.

31. Compound of the formula VI 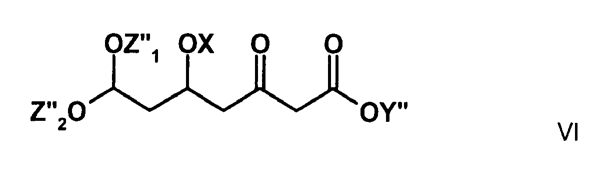 or a salt thereof, wherein Z"₁ is a hydroxy protecting group, Z"₂ is a hydroxy protecting group, X is H or a hydroxy protecting group and Y" is a carboxy protecting group.

32. Compound of the formula VIa 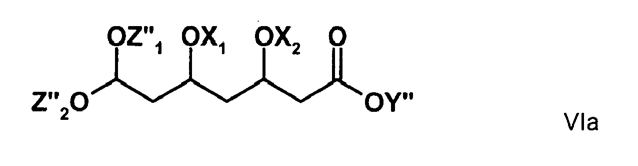 or a salt thereof, wherein Z"₁ is a hydroxy protecting group, Z"₂ is a hydroxy protecting group, X₁ and X₂ can be independent from each other a hydroxy protecting group or X₁ and X₂ taken together may form a ring with the oxygen and intermediate carbon atoms to which they are attached and Y" is a carboxy protecting group.

33. Compound of the formula VIb 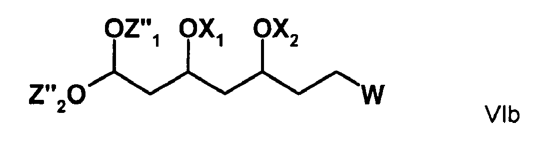 wherein Z"₁ is a hydroxyl protecting group, Z"₂ is a hydroxyl protecting group, X₁ and X₂ can be independent from each other a hydroxyl protecting group or X₁ and X₂ taken together may form a ring with the oxygen and intermediate carbon atoms to which they are attached and W is NH₂, N₃, OH, OW₁ or a leaving group and W₁ is a hydroxyl protecting group.

34. Compound of the formula IX 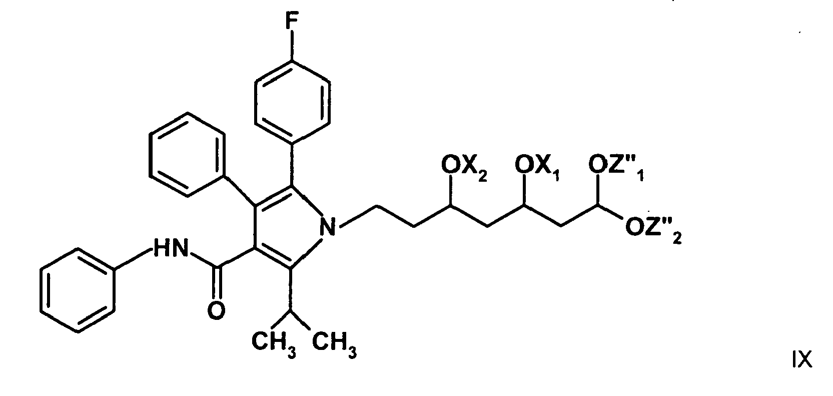 wherein Z"₁ is a hydroxyl protecting group, Z"₂ is a hydroxyl protecting group, X₁ and X₂ can be independent from each other a hydroxyl protecting group or X₁ and X₂ taken together may form a ring with the oxygen and intermediate carbon atoms to which they are attached.

35. Compound of the formula XVI

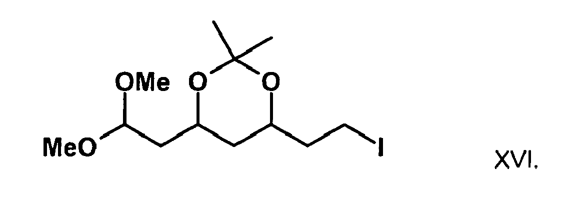

36. Compound of the formula XVIII

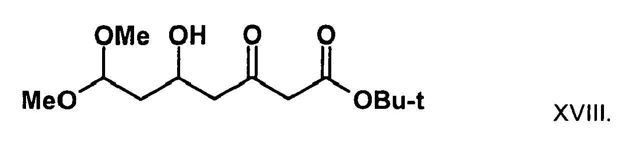

37. Compound of the formula XIX

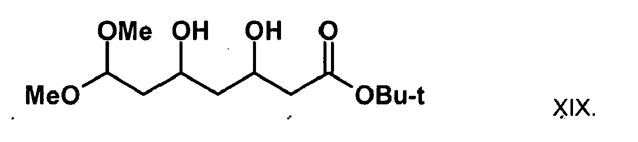

38. Compound of the formula XX

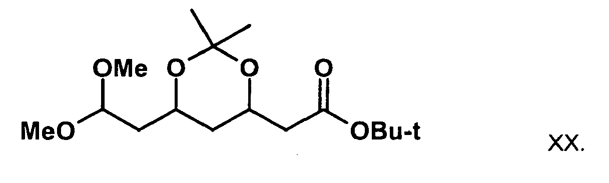

39. Compound of the formula XXI

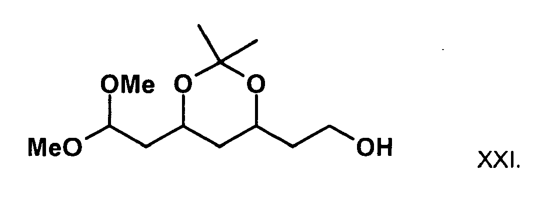

40. Compound of the formula XXII

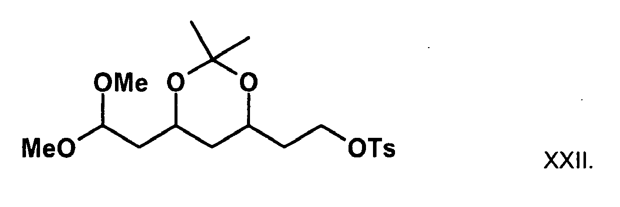

41. Compound of the formula XXIII

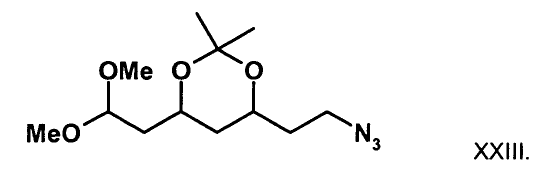

42. Compound of the formula XXIV

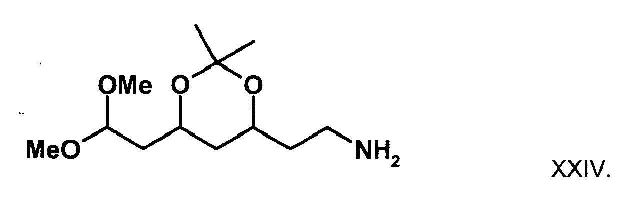

43. Use of a compound of any of claims 23-42 for the preparation of atorvastatin.
